(19) Europäisches Patentamt
European Patent Office
Office européen des brevets

(11) Numéro de publication: **0 464 077 B1**

(12) # FASCICULE DE BREVET EUROPEEN

(45) Date de publication de fascicule du brevet: **02.02.94**

(51) Int. Cl.$^5$: **A61K 9/127**, A61K 47/48

(21) Numéro de dépôt: **90904856.3**

(22) Date de dépôt: **16.03.90**

(86) Numéro de dépôt internationale :
**PCT/FR90/00176**

(87) Numéro de publication internationale :
**WO 90/11069 (04.10.90 90/23)**

(54) **PROCEDE DE PREPARATION DE COMPOSE LIGAND-RECEPTEUR.**

(30) Priorité: **20.03.89 FR 8903628**

(43) Date de publication de la demande:
**08.01.92 Bulletin 92/02**

(45) Mention de la délivrance du brevet:
**02.02.94 Bulletin 94/05**

(84) Etats contractants désignés:
**BE CH DE ES FR GB IT LI LU NL**

(56) Documents cités:
**EP-A- 0 028 917**
**EP-A- 0 285 178**
**WO-A-86/05789**
**WO-A-87/05300**
**FR-A- 2 609 397**

**Database STN, (Karlsruhe) File cas,vol. 106, AN 193579j, 8 June 1987, K. Toda et al.**

**Biochimica et Biophysica Acta, vol. 946, 1988, Elsevier Science Publishers B.V., J. Haensler e tal., pp. 95-105**

(73) Titulaire: **PARFUMS CHRISTIAN DIOR**
**30 avenue Hoche**
**F-75008 Paris(FR)**

(72) Inventeur: **DENIS, Alain**
**97, Route de Nestin**
**F-45450 Fay-aux-Loges(FR)**
Inventeur: **KIEDA, Claudine**
**2, rue de la Tour**
**F-45000 Orleans(FR)**
Inventeur: **MONSIGNY, Michel**
**341, rue des Bouvreuils**
**F-45590 S.-Cyr-en-Val(FR)**
Inventeur: **PERRIER, Pierre**
**22, rue Jousselin**
**F-45000 Orleans(FR)**
Inventeur: **REDZINIAK, Gérard**
**101, rue des Fauvettes**
**F-45590 S.-Cyr-en-Val(FR)**

(74) Mandataire: **Portal, Gérard et al**
**Cabinet Beau de Loménie**
**158, rue de l'Université**
**F-75340 Paris Cédex 07 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne essentiellement un procédé pour fixer un produit sur la membrane d'un kératinocyte au moyen d'une liaison ligand-récepteur, procédé de préparation d'un tel produit, produit obtenu, composition cosmétique ou pharmaceutique le contenant et son procédé de préparation.

On sait depuis longtemps qu'il existe des processus de reconnaissance des cellules, par exemple vis-à-vis d'autres cellules ou de substances telles que des protéines. Ces processus agissent par le biais d'une réaction entre un ligand et un récepteur présent à la surface des cellules.

On connaît par exemple la reconnaissance d'antigènes de surface par des anticorps monoclonaux, ce phénomène étant mis à profit dans des applications thérapeutiques ou diagnostiques. On peut citer par exemple le document FR-A1-2 606 034 qui décrit des anticorps capables de reconnaître une nouvel le protéine de surface, et qui peuvent être utilisés notamment comme réactifs dans les techniques basées sur la réaction antigène-anticorps.

On connaît également des processus de reconnaissance impliquant des sucres. Ceux-ci sont reconnus de manière spécifique par des récepteurs membranaires. Toutefois, les sucres reconnus ne sont pas les mêmes d'un type de cellule à un autre. On sait par exemple que les hépatocytes de rat reconnaissent spécifiquement le galactose (Ashwell G. et Morell A.G., Advan. Enzymol. (1974) 41, 99-128) alors que les fibroblastes humains reconnaissent le mannose-6-phosphate.

A partir de ces connaissances acquises, on a cherché à mettre au point des systèmes de délivrance ciblée de médicaments permettant d'atteindre dans l'organisme les sites sur lesquels on souhaite agir. On a utilisé en particulier pour cela des liposomes.

Depuis leur découverte par BANGHAM (J. Mol. Biol., 13, 238- 252, (1965), les liposomes ont été utilisés comme vecteurs de principe actif médicamenteux ou autres (voir par exemple FR-A-2 221 122 ou FR-A-2 415 460 ou FR-A-2 540 381).

Le document FR-A-2 609 397 concerne l'utilisation d'une substance ou composition de nature glucidique comme principe actif d'une composition dermatologique, ou cosmétologique, ou pharmaceutique, ou stimulante cellulaire, dans le but de renforcer le potentiel bioénergétique cellulaire ou tissulaire (voir revendication 1) ou pour améliorer le confort cutané, grâce à leur effet hydratant, adoucissant, assouplissant (page 3, ligne 33 à page 4, ligne 10 ; où il est même indiqué une action anti-inflammatoire).

Ce document concerne donc un enseignement général qui n'est pas relatif au problème particulier des kératinocytes.

Le document PCT/WO 87/05300 BIOCOMPATIBLES LTD est relatif à un procédé pour préserver un matériau ayant une structure dépendante de l'eau, comprenant la mise en contact du matériau avec une solution aqueuse d'un composé polyhydroxy et ensuite l'enlèvement de l'eau de ce matériau, ceci dans le but de permettre une conservation à long terme de matériaux biologiques comme l'hémoglobine, des erythrocytes, des liposomes et des cellules (voir page 1, lignes 1 à 4).

Ce document concerne le problème du séchage de protéines à partir de solutions aqueuses contenant des saccharides pour éviter des dégradations chimiques et physiques (voir page 3, 2ème paragraphe complet).

Il a été proposé plus récemment de donner une spécificité aux liposomes (voir BARBET J. et al. dans "Monoclonal Antibody covalently coupled to liposomes : Specific Targeting to Cells" dans J. Supramol. Struct. Cell. Biochem. ; 16, 243-258, (1981) ainsi que LESERMAN L.D. et al "Targeting to Cells of fluorescent Liposomes covalently coupled with Monoclonal Antibodies or protein A" dans Nature, 228, 602-604 (1980).

Par ailleurs, le document WO 88/00474 décrit une structure de membrane lipidique comprenant une vésicule lipidique encapsulant un médicament, et un ligand comportant un résidu spécifique des récepteurs hépatobiliaires, afin d'aboutir à un pilotage de la structure vers les hépatocytes avec une efficacité exceptionnelle.

Le document EP-A-0 028 917 est relatif à des vésicules lipidiques portant des surfaces hydrocarbonées comme véhicules lymphatiquement ciblés dans des buts thérapeutiques et de diagnostic. Ce document ne concerne pas le ciblage des kératinocytes.

Le document EP-A-0 285 178 est relatif à des dérivés de mannobiose utiles comme un composant de modification des préparations pharmaceutiques, comme des liposomes, ayant une affinité spécifique pour les cellules de Kupffer du foie.

L'abrégé de la bande de données STN de KARLSRUHE, vol. 106, du 8 juin 1987, résumé 193579j, cite un article de TODA dans Journal of Investment Dermatology, 88(4), 412-417, qui traite de la culture de kératinocytes humains, fraîchement isolés, sur des substrats liés à un ligand différent, incluant de la fibronectine, du collagène ou une membrane de structure.

Ce document va dans une direction totalement différente de celle qui est l'objet de l'invention.

En effet, ce document préconise une culture spécifique des kératinocytes pour les activer en vue d'activer leur capacité à se multiplier sur tous les substrats revêtus de ligands.

Cet article démontre que l'invention décrite ci-après n'était nullement évidente à l'homme de l'art.

Enfin, la demande de brevet PCT/WO 86/05789 BIOCARB concerne également des dérivés hydrocarbonés dans le cadre d'un ciblage spécifique dans un but thérapeutique ou de diagnostic, ce ciblage étant réalisé sur des molécules d'organes différents de mammifères incluant l'homme (voir page 1, lignes 4 à 8).

Il apparaît des buts indiqués à la page 2 que l'invention décrite dans ce document concerne la détection de bactéries pathogènes par des récepteurs particuliers.

Ceci ne concerne donc pas du tout le problème du ciblage des kératinocytes, objet de l'invention décrite ci-après.

En ce qui concerne la peau, on constate qu'il y a un intérêt évident à ce que les produits dermatologiques et surtout cosmétiques, appliqués localement, se fixent préférentiellement et spécifiquement dans l'épiderme. La fixation sur les récepteurs membranaires des kératinocytes, qui sont les cellules essentielles de l'épiderme, constitue une solution à ce problème.

Or Jusqu'à présent, on ne connaissait pas de récepteur de sucre à la surface des kératinocytes.

Selon la présente invention, on a découvert de manière totalement inattendue que l'on pouvait fixer un produit sur la membrane d'un kératinocyte au moyen d'une liaison ligand-récepteur lorsque l'on utilise un produit porteur d'au moins un ligand constitué par un résidu osidique choisi parmi le rhamnose, le galactose et le galactose-6-phosphate. On a en effet découvert, de manière inattendue, que le rhamnose, le galactose et le galactose-6-phosphate sont des ligands spécifiques des sites récepteurs de la membrane des kératinocytes. On a en outre observé que l'α-L-rhamnose et l'α-D-galactose-6-phosphate présentent une spécificité nettement plus importante que l'α-D-galactose, et sont de ce fait préférés par rapport à ce dernier.

En outre, il a été également mis en évidence que le produit porteur de ce sucre formant ligand spécifique des récepteurs de la membrane des kératinocytes pouvait se présenter sous diverses formes. Il peut s'agir par exemple d'une particule submicroscopique, telle qu'un liposome ou une nanoparticule polymérique ou encore une molécule ou une macromolécule d'origine naturelle ou synthétique, telle qu'une protéine. En outre, le sucre formant ligand peut être couplé de diverses manières à la surface de ce produit. Il peut être couplé avantageusement par une liaison chimique covalente, de préférence par l'intermédiaire d'un bras espaceur.

Ainsi, la présente invention a pour objet de résoudre le nouveau problème technique consistant en la fourniture d'une solution permettant de fixer spécifiquement un produit sur la membrane des kératinocytes au moyen d'un liaison ligand-récepteur, le cas échéant au détriment d'autres catégories de cellules environnant les kératinocytes.

La présente invention a pour but de résoudre le nouveau problème technique ci-dessus selon un procédé extrêmement simple, rapide, pratique et fiable. La présente invention a encore pour but de résoudre ce nouveau problème technique en fournissant un procédé de sélection d'un produit susceptible de se fixer spécifiquement sur la membrane d'un kératinocyte au moyen d'une liaison ligand-récepteur qui soit particulièrement simple, rapide, pratique et fiable.

La présente invention a encore pour but de résoudre le nouveau problème technique précité en fournissant un procédé de préparation d'un tel produit susceptible de se fixer spécifiquement sur la membrane d'un kératinocyte au moyen d'une liaison ligand-récepteur qui soit aussi simple, rapide, pratique et fiable. La présence invention a encore pour but de résoudre le nouveau problème technique précité en fournissant un produit susceptible de se fixer spécifiquement sur la membrane d'un kératinocyte au moyen d'une liaison ligand-récepteur qui se présente sous une forme particulièrement aisée à manipuler, qui soit fiable et puisse être utilisé rapidement sans nécessiter d'étapes particulières. La présente invention a encore pour but de résoudre le nouveau problème technique précité en fournissant une composition cosmétique ou pharmaceutique, notamment dermatologique et son procédé de préparation, incorporant un tel produit spécifique qui présente tous les avantages techniques déterminants précités relatifs à la fiabilité de la fixation spécifique sur la membrane d'un kératinocyte au moyen d'une liaison ligand-récepteur selon une méthode rapide, pratique.

La présente invention résout le problème technique précité en fournissant les solutions précitées d'une manière extrêmement simple, utilisable à l'échelle industrielle.

Ainsi, selon un premier aspect, la présente invention fournit un procédé pour fixer un produit sur la membrane d'un kératinocyte au moyen d'une liaison ligand-récepteur, caractérisé en ce que l'on utilise un produit constitué d'un produit de base couplé à au moins un ligand constitué par un résidu osidique accessible aux récepteurs membranaires, ledit résidu osidique étant choisi parmi le rhamnose, le galactose

et le galactose-6-phosphate, et de préférence parmi l'α-L-rhamnose et l'α-D-galactose-6-phosphate. Il est à noter que l'invention inclut toutes les formes isomères du rhamnose et du galactose.

Selon une caractéristique avantageuse, le ligand est couplé à la surface du produit de base par une liaison chimique covalente.

Selon une autre caractéristique avantageuse du procédé selon l'invention, le ligand est couplé à la surface du produit de base précité par l'intermédiaire d'un bras espaceur tel que le résidu d'un réactif hétérobifonctionnel ou un groupe de plusieurs sucres, tel que le groupe (1-4)-O-β-D-glucopyranosyl-(1-6)-O-β-D-glucopyranosyle. De tels bras espaceurs sont bien connus à l'homme de l'art qui pourra se reporter par exemple à l'article de J.S. Slama et R.R. Rando dans Biochemistry, 1980, 19, 4595-4600.

Selon une autre caractéristique particulièrement avantageuse du procédé selon l'invention, le produit de base précité est une particule submicroscopique telle qu'un liposome ou une nanoparticule polymérique.

Selon encore une autre caractéristique particulièrement avantageuse du procédé selon l'invention, le produit ou le produit de base précité est une molécule ou une macromolécule d'origine naturelle ou synthétique, telle que

- un asiaticoside, contenant l'α-L-rhamnose extrait en particulier de la plante Centella asiatica, disponible par exemple chez Inverni Della Beffa,
- un digalactosyldiglycéride, obtenu notamment à partir d'un extrait de farine de blé, disponible par exemple chez Sigma,
- une néoglycoprotéine obtenue par exemple en combinant une albumine de sérum (SA) par exemple bovin ou humain à un sucre précité.

Selon une autre caractéristique du procédé selon l'invention, le produit ou le produit de base précité est ou contient une substance d'intérêt cosmétique ou pharmaceutique, notamment dermatologique, telle qu'un agent modulateur du métabolisme des cellules de la peau.

Selon un deuxième aspect, la présente invention fournit aussi un procédé pour sélectionner un produit destiné à être fixé sur la membrane d'un kératinocyte au moyen d'une liaison ligand-récepteur, caractérisé en ce que l'on choisit, parmi un ensemble de produits, ceux constitués d'un produit de base couplé à au moins un ligand constitué par un résidu osidique accessible aux récepteurs, ledit résidu osidique étant choisi parmi le rhamnose, le galactose ou le galactose-6-phosphate, de préférence l'α-L-rhamnose et l'α-D-galactose-6-phosphate.

Selon un troisième aspect, la présente invention fournit encore un procédé de préparation d'un produit destiné à être fixé sur la membrane d'un kératinocyte au moyen d'une liaison ligand-récepteur, caractérisé en ce que l'on couple un produit de base correspondant à au moins un ligand constitué par un résidu osidique accessible aux récepteurs membranaires, ledit résidu osidique étant choisi parmi le rhamnose, le galactose et le galactose-6-phosphate, de préférence l'α-L-rhamnose et l'α-D-galactose-6-phosphate.

Selon une caractéristique particulièrement avantageuse, le ligand précité est couplé au produit de base par une liaison chimique covalente. Le couplage peut être réalisé conformément à la méthode générale décrite par MacBroom dans Methods in Enzymology (1972) 28, 212-222 ou encore celle décrite par F.J. Martin et D. Papahadjopoulos dans The Journal of Biological Chemistry (1982), vol 257, n° 1, pages 286-288.

Selon une autre caractéristique avantageuse du procédé selon l'invention, le ligand précité est couplé au produit de base par l'intermédiaire d'un bras espaceur tel que précédemment défini.

Selon encore une autre caractéristique avantageuse du procédé selon l'invention, le produit de base est une particule submicroscopique telle qu'un liposome ou une nanoparticule polymérique.

Selon encore une autre caractéristique du procédé selon l'invention, le produit de base précité est une molécule ou une macromolécule d'origine naturelle ou synthétique, telle qu'une protéine, par exemple une albumine de sérum.

Selon encore une autre caractéristique avantageuse du procédé selon l'invention, le produit ou le produit de base précité est ou contient une substance d'intérêt cosmétique ou pharmaceutique, notamment dermatologique, telle qu'un agent modulateur du métabolisme des cellules de la peau.

L'invention, selon un quatrième aspect, couvre aussi un produit destiné à être fixé sur la membrane d'un kératinocyte au moyen d'une liaison ligand-récepteur, caractérisé en ce qu'il est susceptible d'être obtenu selon l'un des procédés précédemment définis. L'invention couvre aussi les nouveaux produits susceptibles de se fixer sur la membrane d'un kératinocyte au moyen d'une liaison ligand-récepteur, caractérisé en ce qu'ils comprennent au moins un ligand constitué par un résidu osidique accessible aux récepteurs membranaires, ledit résidu osidique étant choisi parmi le rhamnose, le galactose et le galactose-6-phosphate et de préférence l'α-L-rhamnose et l'α-D-galactose-6-phosphate, couplé à un produit de base, par exemple choisi parmi le groupe constitué par une particule submicroscopique telle qu'un liposome ou une nanoparticule polymérique ; une molécule ou une macromolécule d'origine naturelle ou synthétique,

telle qu'une protéine, en particulier une albumine de sérum ; une substance d'intérêt cosmétique ou pharmaceutique, notamment dermatologique, telle qu'un agent modulateur du métabolisme des cellules de la peau. Selon une caractéristique avantageuse, ce couplage a lieu par l'intermédiaire d'un bras espaceur tel que le résidu d'un réactif hétérobifonctionnel ou un groupe de plusieurs sucres, tel que le groupe (1-4)-O-$\beta$-D-glucopyranosyl-(1-6)-O-$\beta$-D-glucopyranosyle.

Selon un cinquième aspect, la présente invention fournit encore une composition cosmétique ou pharmaceutique, notamment dermatologique, caractérisée en ce qu'elle contient au moins un produit tel qu'obtenu par le procédé précédemment décrit ou tel que précédemment défini.

Selon une caractéristique avantageuse de l' invention, la composition précitée est destinée à tout soin ou tout traitement intéressant les kératinocytes, par exemple l'amélioration de la régénération cutanée, le traitement du psoriasis ou la repousse des cheveux.

Enfin, l'invention concerne encore un procédé de préparation d'une composition cosmétique ou pharmaceutique, notamment dermatologique, caractérisée en ce que l'on associe à un excipient, véhicule ou support cosmétiquement ou pharmaceutiquement acceptable, au moins un produit tel qu' obtenu par l'un des procédés précédemment décrits, ou tel que précédemment définis. De même, l'invention couvre encore un procédé de soin ou de traitement intéressant les kératinocytes, en vue par exemple de la régénération cutanée, du traitement du psoriasis ou de la repousse des cheveux, caractérisé en ce qu'on applique le produit tel que précédemment obtenu par l'un des procédés précédemment décrits, ou tel que précédemment définis, en une quantité cosmétiquement ou thérapeutiquement efficace, avantageusement alors que celui-ci est associé à un véhicule, support ou excipient cosmétiquement ou pharmaceutiquement acceptable.

On conçoit que, grâce à l'invention, on obtienne un ciblage cellulaire d'un produit que l'on peut ainsi concentrer spécifiquement au niveau des Kératinocytes grâce à la présence d'au moins un ligand spécifique constitué par un résidu osidique choisi parmi le rhamnose, le galactose et le galactose-6-phosphate, de préférence l'$\alpha$-L-rhamnose et l'$\alpha$-D-galactose-6-phosphate.

Dans le cadre de la présente invention, le produit de base auquel est couplé le ligand spécifique constitué par le résidu osidique peut être quelconque.

On peut, par exemple, utiliser une protéine à laquelle on couple par voie de synthèse l'un des sucres précités.

Selon un mode de réalisation avantageux de l'invention, le nombre de résidus osidiques tels que précédemment définis portés par la protéine est d'au moins vingt environ.

On peut également utiliser comme produit de base une particule submicroscopique telle qu'une vésicule lipidique pouvant se présenter sous forme de liposome.

Dans le cadre de la présente invention, le terme "lipidique" couvre toutes les substances comprenant une chaîne carbonée dite grasse, généralement supérieure à 5 atomes de carbone, cette substance étant habituellement dénommée "lipide".

Selon l'invention, on utilise à titre de lipide pour former le liposome précité, des lipides amphiphiles, c'est-à-dire constitués de molécules possédant un groupe hydrophile indifféremment ionique ou non ionique et un groupe lipophile, ces lipides amphiphiles étant susceptibles de former des phases lamellaires lipidiques ou des liposomes en présence d'une phase aqueuse.

En particulier, on peut citer parmi ces lipides : les phospholipides, les phosphoaminolipides, les glycolipides, les alcools gras polyoxyéthylénés, les esters de polyols éventuellement polyoxyéthylénés. De telles substances sont par exemple constituées par une lécithine d'oeuf ou de soja, une phosphatidylsérine, une sphyngomyéline, un cérébroside ou un stéarate de polyglycérol oxyéthyléné.

Selon une caractéristique avantageuse de la présente invention, en ce qui concerne les liposomes, la proportion, en pourcentage molaire des dérivés osidiques selon l'invention par rapport à l'ensemble des molécules lipidiques formant la bicouche du liposome est d'au moins 10%, et de préférence d'au moins 15 % par rapport à l'ensemble des lipides amphiphiles du liposome.

Il est également préféré selon l'invention que les liposomes soient formulés de manière à présenter une forte microviscosité. Ceci peut par exemple être obtenu en utilisant des lipides amphiphiles dont la température de transition est supérieure à environ 37 °C et de préférence 41 °C, notamment les phospholipides ou acides gras saturés, par exemple la dipalmitoyle phosphatidylcholine.

On remarquera également qu'en raison de l'hydrophilie des résidus osidiques, ceux-ci se répartissent à l'extérieur de la bicouche lipidique des liposomes et sont orientés les uns vers l'intérieur, les autres vers l'extérieur du liposome. Il a d'ailleurs été observé que plus les liposomes sont de petite taille, plus le nombre de résidus osidiques orientés vers l'extérieur est important. Ainsi, il s'en trouve un nombre suffisant pour être facilement accessibles aux récepteurs des kératinocytes. On comprendra que cette accessibilité est encore meilleure lorsque les résidus osidiques sont placés à l'extrémité d'un bras espaceur.

On peut également utiliser selon l'invention comme produit de base une nanoparticule polymérique, généralement de dimension inférieure au micromètre, de préférence des nanoparticules polymériques comportant des groupes $NH_2$, des fonctions alcools ou des fonctions thiols permettant un couplage aisé avec le résidu osidique selon l'invention. On choisit de préférence des nanoparticules biodégradables, telles que polylactates, bien connues à l'homme de l'art. De telles particules particulières comportant des groupes $NH_2$ sont disponibles dans le commerce, par exemple chez Interfacial Dynamics Corporation (Portland, Oregon-USA).

Selon une caractéristique avantageuse de la présente invention, les résidus osidiques couplés à la surface desdites nanoparticules polymériques sont choisis parmi les résidus de α-D-galactose, α-L-rhamnose et α-D-galactose-6-phosphate.

On peut utiliser avantageusement pour encapsuler les substances actives, des nanoparticules poreuses.

Suivant un mode avantageux de réalisation de l'invention, les produits de base, tels que les liposomes ou les nanoparticules précités, contiennent une ou plusieurs substances d'intérêt cosmétique ou pharmaceutique, notamment dermatologique, telle qu'un agent modulateur du métabolisme des cellules de la peau, par exemple des peptides obtenus par hydrolyse de protéines, des modulateurs d'activité enzymatique, par exemple une xanthine telle que la théophylline.

Le produit de base utilisable selon l'invention peut également être la substance active elle-même d'intérêt cosmétique ou pharmaceutique, notamment dermatologique.

D'autres buts, caractéristiques et avantages de l'invention apparaîtront clairement à la lumière de la description explicative qui va suivre à l'aide de plusieurs exemples de l'invention donnés simplement à titre d'illustration et qui ne sauraient donc en aucune façon limiter la portée de l'invention. Dans les exemples tous les pourcentages sont donnés en poids sauf indication contraire.

Exemple 1 selon l'invention

Préparation de liposomes porteurs de résidus rhamnosylés

A. **Couplage de l'α-L-rhamnose sur un phospholipide**

Le couplage entre une molécule de dipalmitoylphosphatidyle éthanolamine (DPPE) et une molécule d'α-L-rhamnose (Rha) est réalisé par réaction du p-phénylisothiocyanate-α-L-rhamnopyranoside (PPITC-α-L-rhamnopyranoside) sur la fonction amine de la DPPE.

Le PPITC-α-L-rhamnopyranoside est préparé à partir du p-aminophényl-α-L-rhamnoside, par réaction avec le thiophosgène, selon le procédé décrit par C.R. McBroom et al. dans Methods in Enzymology (1972) 28 212-222.

La DPPE du commerce est dissoute mole à mole avec le PPITC-α-L-rhamnopyranoside à 50 °C dans un mélange constitué d'un tampon bicarbonate (0,1 M, pH 9,5) et d'éthanol. La température est maintenue à 50 °C pendant 3 h, sous agitation. Le milieu réactionnel est ensuite refroidi à 4 °C environ, et on centrifuge.

Le solide obtenu, après lavage à l'eau distillée, est lyophilisé. On obtient ainsi une DPPE rhamnosylée (DPPE-Rha).

B. **Préparation des liposomes**

On pèse :

| | |
|---|---|
| - Dipalmitoylphosphatidylcholine (DPPC) | 48,6 mg |
| - Cholestérol (chol) | 25,6 mg |
| - Dicétylphosphate (DCP) | 9,1 mg |
| - Produit selon l'invention = DPPE-Rha | 16,7 mg |

Après avoir mélangé les composés précités dans un ballon à fond plat, par exemple de 100 ml, on les solubilise avec un mélange chloroforme-méthanol 7/1, conformément à la méthode du ballon rotatif décrite par Bangham dans J. Mol. Biol., 13, 238-252, (1965). On évapore ensuite les solvants à 55 °C sous pression réduite. On peut éliminer les traces des solvants par un courant d'azote sur le film "gras" formé sur la paroi du ballon.

On ajoute ensuite 10 ml de la solution aqueuse à encapsuler dans les liposomes formés à partir de la phase lipidique précitée. Cette solution aqueuse peut par exemple contenir un marqueur fluorescent constitué par exemple le sel potassique de 5-(6)-carboxyfluoresceine à raison de 8 g pour 40 ml d'eau distillée.

Comme marqueur fluorescent, on peut également utiliser de manière équivalente la calcéine bleue.

Après avoir ajouté 10 ml de la solution aqueuse à encapsuler ci-dessus, on place sous agitation magnétique de 15 à 24 h à l'abri de la lumière.

On observe au microscope optique la formation de grosses vésicules lipidiques.

On récupère le contenu du ballon dans un récipient de 30 ml refroidi dans la glace fondante.

De préférence, on soumet la suspension à sonication trois fois à 2 min à 4 ° C à une puissance de 200 W, ce qui permet de former des liposomes de taille sensiblement homogène, de dimension submicroscopique.

On peut conserver la suspension liposomale ainsi obtenue à 4 ° C.

Selon un mode de réalisation préféré, on réalise une purification des liposomes en les séparant de la phase aqueuse non encapsulée par une filtration sur gel, comme cela est bien connu selon le protocole classique de purification des liposomes.

Exemple 2 selon l'invention

Préparation de liposomes porteurs de résidus rhamnosylés par l'intermédiaire de bras espaceurs

A. **Préparation du rhamnose porteur d'un bras espaceur**

On prépare, comme indiqué ci-dessous, un "sucre activé" de de formule (I) constitué d'un résidu α-L-rhamnopyranosyle, dont le groupe OH situé sur le premier carbone est substitué par une chaîne linéaire terminée par un groupe SH.

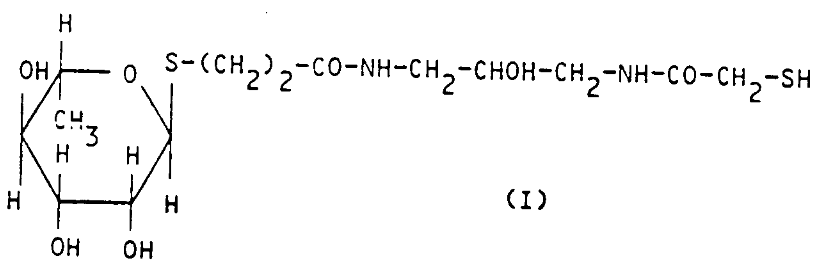

(I)

Pour ce faire, on procède aux étapes successives suivantes :

* Synthèse du 2,3,4-tri-O-acétyl-α-L-bromorhamnopyranside (2)

  Le dérivé (2) est obtenu selon KEMPEN H.J.M. et al., J.Med.Chem. (1984) 27 1306-1312, par action du bromure de phosphore $PBr_3$ (7 eq.) et de l'eau (50 eq) sur l'α-L-rhamnose-1 ,2, 3,4-tétra-O-acétylé (1), dans l'anhydride acétique sous agitation à 0 ° C. Le produit (2) est recristallisé dans un mélange diisopropyléther/hexane.

* Synthèse du bromhydrate de 2-S-(2,3,4-tri-O-acétylul-α-L-rhamnopyranosyl)-2-thiopseudourée (3).

  Le dérivé (3) est obtenu selon CHIPOWSKY S. et al., Carbohyd. Res. (1973) 31 339-346 en chauffant sous reflux et sous argon pendant 3 h, un mélange du dérivé (2) et de thiourée en proportion équimoléculaire dans l'acétone anhydre. Le produit (3) est recristallisé dans l'isopropanol.

* Synthèse du 1-(2-carboxyéthyl)thio-2,3,4-tri-O-acétyl-α-L-rhamnopyranoside (4).

  Le produit (4) est obtenu par réaction équimoléculaire entre le dérivé (3) et l'acide 3-iodopropionique en solution dans un mélange eau/acétone 1:1 en présence de 1,15 eq. de $Na_2CO_3$ et de 2 eq. de $Na_2S_2O_5$. La réaction est suivie par CCM (solvant AcOEt/AcOH/$H_2O$ 8:2:1).

* Synthèse du 1-(2-carboxyéthyl)thio-α-L-rhamnopyranoside (5).

  Le dérivé (5) est obtenu par désacylation de (4) en présence de 4 eq. de triéthylamine et de 3 eq. d'eau dans du méthanol. La réaction est suivie par CCM (solvant AcOEt/AcOH/$H_2O$ 8:2:1) ; elle est complète au bout de 6 jours. Le produit de désacétylation est isolé du milieu réactionnel par passage sur colonne Dowex 1x2 ($HCOO^-$).

* Synthèse du chlorhydrate du 1-(2-(3-amino-2-hydroxy-propylaminocarbonyl)-éthyl)thio-α-L-rhamnopyranoside (6).

  0,5 g de (5) (1,8 mmole) et 0,8 g de 1,3-diaminopropanol-2 (9 mmoles) sont dissous dans 20 ml d'eau. Le pH de la solution est ajusté à 5,5 (HCl) et on ajoute alors 1,1 g de 1-éthyl-3-(3-diméthylaminopropyl)carbodiimide (5,4 mmoles). On laisse réagir 24 h sous agitation à température ambiante. Le produit (6) est isolé du milieu réactionnel par passage sur Dowex 50x2 ($Na^+$) (éluants : HCl 0,33 N pour décrocher les monoamines et HCl 2 N pour décrocher les diamines). Finalement, on obtient le produit (6).
* Synthèse de l'amide entre le composé (6) et l'acide acétyl-S-thioglycolique (7).

  Le produit (7) est obtenu par réaction équimoléculaire entre le dérivé (6) et le succinimidyl-S-acétylthioacétate (SATA, synthèse selon Ducan et al. Anal. Biochem. (1983) 132 68-73) en présence de triéthylamine (1 eq) dans le diméthylformamide anhydre. Le produit (7), obtenu est utilisé dans la suite des opérations sans purification particulière.
* Déprotection de la fonction thiol du composé (7) : synthèse du dérivé de formule (I)

  Sur le produit (7) (1 eq.) on génère sous argon une fonction thiol à l'aide d'un fort excès d'hydroxylamine (50 eq.), dans une solution aqueuse 0,15 M en EDTA, pH = 7,5. La réaction est suivie par dosage spectrophotométrique des thiols en utilisant le réactif d'Ellman selon la méthode de P.W. RIDDLES et al., Anal. Biochem. (1978) 94 75-81. Elle est complète au bout de 1 h. Le produit de formule (I) obtenu est utilisé rapidement pour le greffage sur les liposomes.

B**. Préparation de liposomes porteurs de groupes maléimide.**

Ces liposomes sont préparés selon une méthode classique, par exemple comme dans l'exemple 1. La composition de la phase lipidique est un mélange en proportion molaire 10:2:7 des composés suivants :

- dipalmitoylphosphatidylcholine (DPPC)
- N-[4-(p-maléimidophényl)butyrylγ-dipalmitoylphosphatidyléthanolamine (MPB-DPPE)
- cholestérol (chol).

Le MPB-DPPE est un phospholipide porteur d'un groupe maléimide, préparé selon le procédé décrit par Martin F.J. et Papahadjopoulos D., J. Biol. Chem. (1982) 257 286-288, par réaction de la dipalmitoylphosphatidyléthanolamine sur le succinimidyl-4-(p-maléimidophényl)butyrate (SMPB).

C**. Préparation de liposomes rhamnosylés.**

On procède selon la méthode décrite par Martin F.J. et Papahadjopoulos D. (référence citée ci-dessus).

Ainsi, 6 μmoles du composé de formule (I) préparé à l'étape A sont introduites dans 0,67 ml de suspension aqueuse de liposomes préparés à l'étape B, tamponnée à pH 6,5.

Le mélange est placé sous atmosphère d'argon, sous agitation douce pendant 12 h à température ordinaire.

La fonction thiol du composé de formule (I) se fixe alors par réaction d'addition sur la double liaison du groupe maléimide.

On obtient ainsi des liposomes comportant en surface des résidus rhamnosylés situés à l'extrémité d'une chaîne d'atomes (bras espaceur) correspondant à la chaîne du composé de formule (I).

Exemple 3 selon l'invention

Préparation de liposomes porteurs de résidus rhamnosylés par l'intermédiaire de bras espaceurs

La composition de la phase lipidique est un mélange en proportion molaire 4:4:1:1 des composés suivants :

- Dipalmitoylphosphatidylcholine (DPPC)
- Cholestérol (chol)
- Dicétylphosphate (DCP)
- Asiaticoside (C.A.)

L'asiaticoside est extrait de la plante Centella asiatica, disponible par exemple chez Inverni della Beffa, de formule L-asiatate de [ (O-α-L-rhamnopyranosyl-(1-4)-O-β-D-glucopyranosyl-(1-6)γ-O-β-D-glucopyranose (PM = 959 g).

Les constituants de la phase lipidique ci-dessus sont dissous à la concentration de 10 % environ dans un mélange 80:20 en volume de dichlorométhane et de méthanol.

La solution organique est atomisée à 60°C environ selon le procédé décrit dans le document EP-B1-0087993 pour obtenir une poudre.

Cette poudre est ensuite dispersée par agitation dans la solution aqueuse à encapsuler, à raison de 1 partie de poudre pour 100 parties de solution aqueuse. Cette solution peut par exemple contenir un marqueur fluorescent tel que la 5-(6) carboxyfluorescéine, comme dans l'exemple 1.

Il se forme ainsi une suspension de liposomes qui est de préférence homogénéisée, par exemple au moyen d'un homogénéiseur sous pression comme décrit dans le document EP-B1-0107559.

On obtient ainsi une suspension de liposomes comportant en surface des résidus rhamnosylés, reliés à la bicouche par des bras espaceurs constitués du résidu (1-4)-O-β-D-glucopyranosyl-(1-6)-O-β-D-glucopyranosyle.

Exemple 4 selon l'invention

Préparation de liposomes porteurs en surface de résidus galactosylés

On procède comme à l'exemple 1, si ce n'est que l'on utilise le digalactosyldiglycéride, extrait de farine de blé, disponible par exemple chez Sigma et comportant à l'extrémité de la molécule deux résidus galactosylés, à la place du produit selon l'invention utilisé à l'exemple 1.

Exemple 5 selon l'invention

Préparation de nanoparticules fluorescentes comportant en surface des résidus rhamnosylés

On peut se procurer dans le commerce des nanoparticules Sous forme de billes de diamètre moyen de 0,5 µm environ constituées principalement de polystyrène, et possédant en surface des groupes $NH_3^+$. De telles nanoparticules sont disponibles par exemple chez Interfacial Dynamics Corporation (I.D.C., Portland, Oregon, USA).

Il est donc possible, en opérant selon l'étape A de l'exemple 1, de coupler des résidus rhamnosylés sur ces nanoparticules.

Pour cela, on fait réagir, dans 5 ml de tampon bicarbonate à pH 9,5, 0,6 g de ces billes avec 9 µmoles de α-L-rhamnopyranoside phénylisothiocyanate sous agitation douce, pendant 5 h à 4°C.

Les billes sont ensuite recueillies, puis rincées deux fois avec du tampon PBS.

On obtient ainsi des nanoparticules comportant en surface des résidus rhamnosylés.

Exemple 6 selon l'invention

Préparation d'une néoglycoprotéine portant des résidus rhamnosylés

On utilise dans le cas présent une protéine, par exemple de l'albumine de sérum bovin, BSA, à laquelle on couple une vingtaine d'unités de rhamnose en obtenant ainsi une néoglycoprotéine.

Cette néoglycoprotéine appelée BSA-rhamnosylée est obtenue comme suit en s'inspirant de la méthode décrite par MacBroom et al. dans Methods in Enzymology, (1972) 28 212-222.

Pour ce faire, on dissout 250 mg (environ 1 mmole) de PPITC-α-L-rhamnopyranoside préparé selon les indications figurant à l'exemple 1, dans 2 ml d'une solution aqueuse de NaCl 0,15 M et on ajoute 300 g de BSA. Le pH est ajusté à 9,0 par NaOH (1 N). Après 6 h sous agitation à température ambiante, le mélange réactionnel est dialysé pendant 16 h à 4°C contre 2 l de NaCl 0,15 M pH 7,0. La BSA-rhamnosylée est encore purifiée par passage sur colonne Séphadex G25 (10 ml de gel présaturé en BSA, sur colonne de 0,5 x 20 cm). L'absence de toute contamination par des résidus de rhamnose libre est vérifiée en chromatographie sur couche mince où la BSA-rhamnosylée ne migre pas alors que le réactif rhamnosylé présenterait un Rf de 0,32 (chloroforme-MeOH : 1/1). Le dosage des protéines associé au dosage des sucres permet de déterminer un taux de greffage de 10 à 30 résidus de rhamnose par molécule de BSA suivant la préparation considérée.

Exemple 7 selon l'invention

Préparation d'une néoglycoprotéine portant des résidus du galactose-6-phosphate

On prépare dans une première étape le p-aminophényl-6-phospho-α-D-galactopyranoside, à partir du p-nitrophényl-α-D-galactoside du commerce, selon le procédé décrit par G.N. SANDO et E.M. KARSON dans Biochemistry (1980) vol. 19 n° 16 pages 3850-3855, par phosphorylation au moyen du chlorure de phosphoryle, suivie d'une hydrogénation.

Dans une deuxième étape, on prépare le PPITC-6-phospho-α-D-galactopyranoside (PPITC-G) selon la méthode de l'exemple 1, par réaction du thiophosgène sur le composé préparé à la première étape.

Enfin, dans une troisième étape, on laisse incuber 10 mg d'albumine de sérum de boeuf (BSA) en présence de 62 mg de PPITC-G, préparés à l'étape précédente, en opérant comme à l'exemple 6.

On obtient ainsi de la BSA couplée à environ une vingtaine de résidus 6-phospho-α-D-galactoside.

Exemple 8 comparatif

Préparation de liposomes porteurs en surface de résidus glucosylés

Dans cet exemple, on procède à l'exemple 1, si ce n'est que l'on utilise à la place du DPPE-Rha, une molécule d'origine naturelle portant déjà un résidu glucosyle, constitué par le séricoside extrait des racines de Terminalia sericea disponible par exemple chez Inverni Della Beffa.

Exemple 9

Détermination ce la spécificitè des produits selon l'invention vis-à-vis des membranes des kératinocytes

La spécificité des produits selon l'invention porteurs de ligands osidiques vis-à-vis de la membrane des kératinocytes a pu être déterminée grâce a l'emploi de néoglycoprotéines fluorescentes synthétisées par les inventeurs dont on peut observer l' endocytose par diverses techniques. Il faut observer que l' endocytose est d'autant plus importante que la fixation est spécifique au niveau de la membrane celluaire, de sorte que l'observation de l' endocytose mesure indirectement la spécificité de la fixation des produits selon l'invention. Ces diverses techniques comprennent :

a) microscopie de fluorescence, qui permet la visualisation cytochimique qualitative des dérivés fluorescents ;

b) cytofluorimétrie des cellules mises en suspension qui permet, grâce à une analyse cellule par cellule, de préciser la répartition des molécules fluorescentes au sein de l'ensemble de la population cellulaire. En outre, cette technique ouvre la possibilité d'une analyse simultanée de divers paramètres (taille, forme cellulaire, double marquage à l'aide d'anticorps monoclonaux ou étude du cycle cellulaire à l'aide de marqueurs spécifiques de l'ADN ou de l'ARN).

Chacune de ces méthodes est détaillée ci-après après avoir exposé les cultures cellulaires utilisées.

On utilise comme cellules des fibroblastes humains normaux (F26), des kératinocytes humains tumoraux (HSC).

Les milieux de culture proviennent de chez EUROBIO et sont les suivants :

pour les fibroblastes :

le milieu de base de Eagle (MEM), bien connu à l'homme de l'art,

pour les kératinocytes :

un milieu de Ham F12 additionné d'un milieu essentiel minimum de Eagle modifié par Dulbécco (DMEM), également bien connus à l'homme de l'art.

Ces milieux sont complémentés en sérum de veau foetal (10%) et en pénicilline-streptomycine (100 000 UI/l et 0,1 g/l), et en mycostatine (100 000 UI/l).

Les conditions de culture sont les suivantes :

* Pour l'étude par microscopie de fluorescence

Les kératinocytes sont cultivés sur des lamelles de verre qui servent de support.

Pour cela, on place une lamelle au fond de chaque puits d'une plaque de culture de 24 puits type Linbro (diamètre 14 mm).

Culture :

. 20 000 cellules/puits
. volume 200 µl/puits

. incubation : température 32 ° C
humidité 96 %
taux de $CO_2$ 5 %
. temps de culture : 5 jours pour aboutir à la confluence des cellules.

* Pour l'étude en cytométrie en flux

La culture est réalisée en boîtes de Pétri diamètre 60 mm (CORNING).

Culture :

. 200 000 cellules/boîte
. volume 2 ml/boîte
. incubation : température 37 ° C
humidité 96 %
taux de $CO_2$ 5 %
. temps de culture : 7 à 12 jours pour aboutir à la confluence des cellules.

## A. Microscopie de fluorescence

La microscopie de fluorescence permet l'étude des cellules in situ. Il est possible de localiser les parties de la cellule où se fixent préférentiellement les composés fluorescents.

Les substances testées sont des néoglycoprotéines synthétisées à partir de BSA par réaction avec un glycosido-p-phénylisothiocyanate, selon l'exemple 6 ou, le cas échéant, l'exemple 7 lorsque l'on désire un sucre phosphaté. Ensuite, ces néoglycoprotéines sont rendues fluorescentes en les faisant réagir avec du p-phénylisothiocyanate de fluorescéine.

La détection de ces molécules se fera par analyse de fluorescence de la fluorescéine, sachant que celle-ci a une longueur d'onde maximale d'excitation $\lambda_{ex}$ = 490 nm et une longueur d'onde d'émission maximale à 517 nm.

Dans le cas présent, nous utilisons un microscope Zeiss® équipé en "épifluorescence" . L'excitation se fait par une lumière de longueur d'onde sélectionnée par un filtre. De même, un second filtre ne laisse passer la lumière émise que pour des longueurs d'ondes comprises entre 520 et 560 nm.

Les cellules cultivées sur lamelles sont marquées par une néoglycoprotéine de la manière suivante. On remplace dans les boîtes de culture de kératinocytes le milieu par une solution de néoglycoprotéine dans un tampon PBS à la concentration de 1 mg/ml. On laisse incuber pendant 2 h à 32 ° C. On rince ensuite en culture par du tampon PBS à 4 ° C, puis on fixe à l'aide de paraformaldéhyde. On procède de même avec de la BSA fluorescente comme substance témoin.

Sur un deuxième lot de boîtes, on réalise une incubation pendant 1 h 1/2 à 32 ° C puis on ajoute de la Monensine à raison de 1 δg/ml dans le milieu et on poursuit l'incubation pendant 30 min, à 4 ° C, le but de cette opération étant de réhausser le pH intracellulaire afin d'augmenter la sensibilité de la mesure fluorimétrique.

Sur un troisième lot de boîtes, après avoir réalisé l'incubation à 32 ° C pendant 2 h, et la fixation au paraformaldéhyde, on perméabilise la membrane plasmique à l'aide d'un détergent, tel que le Nonidet P40® disponible chez SIGMA. Cette perméabilisation permet de faciliter l'accès des membranes internes aux produits fluorescents, ce qui permet de mettre en évidence des récepteurs de sucre à l'intérieur de la cellule.

L'observation permet de situer la fluorescence émise dans les différentes parties de la cellule :

$V_{mb}$ : vésicules au niveau des membranes
$V_c$ : vésicules dans le cytoplasme
$M_b$ : membrane externe
R : réseau dans le cytoplasme
N : noyau
PN : points autour du noyau

Les résultats de ces observations sont rassemblés au tableau 1 ci-après, pour des néoglycoprotéines se différenciant les unes des autres par la nature des résidus osidiques qu'elles portent.

Il apparaît très clairement que la fluorescence est intense, voire très intense, au niveau de la membrane cellulaire dans le cas du rhamnose, du galactose-6-phosphate, et, dans une nettement moindre mesure, du galactose.

Cette étude montre bien l'existence d'une affinité spécifique des récepteurs membranaires des kératinocytes pour les résidus rhamnose, galactose, galactose-6-phosphate, et plus particulièrement pour l'α-L-rhamnose et l'α-D-galactose-6-phosphate.

Tableau 1

Intensité de fluorescence des kératinocytes marqués par des néoglycoprotéines fluorescentes

| Néoglycoprotéine fluorescente | 32°C (2 h) | 32°C (2 h) + Monensine | Cellules perméabilisées (2 h) avec Nonidet P40® |
|---|---|---|---|
| FBSA (témoin) | - | - | - |
| FBSA Glc α | $V_c^{+}$ | $V_c^{++}$ | $N^{++}$ $PN^{++}$ $R^{+}$ |
| FBSA Glc Nac β | $V_c^{+}$ | $V_c^{++}$ | $N^{++}$ $PN^{++}$ $R^{+}$ |
| FBSA Gal α | $V_c^{\pm}$ $V_{mb}^{+}$ $M_b^{+}$ | $V_c^{+}$ $V_{mb}^{+}$ $M_b^{+}$ | $N^{++}$ $PN^{++}$ $R^{\pm}$ $M_b^{+}$ |
| FBSA Fuc α | - | - | - |
| FBSA Lac β | $V_c^{\pm}$ | $V_c^{\pm}$ | $N^{\pm}$ $PN^{+}$ |
| FBSA Man α | - | - | $PN^{+}$ |
| FBSA Man 6P α | $V_c^{\pm}$ | $V_c^{+}$ | $N^{\pm}$ $PN^{+++}$ |
| FBSA Rha α | $V_c^{++}$ $V_{mb}^{+++}$ $M_b^{+}$ | $V_c^{+++}$ $V_{mb}^{+++}$ $M_b^{+}$ | $N^{+++}$ $PN^{++}$ $M_bR^{++}$ |
| FBSA Gal 6P α | $V_c^{++}$ $V_{mb}^{+++}$ $M_b^{\pm}$ | $V_c^{+++}$ $V_{mb}^{++++}$ $M_b^{\pm}$ | $N^{+++}$ $PN^{++}$ $M_bR^{++}$ |

Intensité de fluorescence :

± très faible
∓ faible
++ moyenne
+++ forte
++++ très forte

Glc : α-D-glucose
GlcNac : N-acétyl-D-glucosamine
Gal : α-D-galactose
Fuc : α-L-fucose
Lac : β-lactose
Man : α-D-mannose
Man 6P : α-D-mannose-6-phosphate
Rha : α-L-rhamnose
Gal 6P : α-D-galactose-6-phosphate

FBSA : albumine de sérum bovin fluorescente

B. **Technique utilisant la cytofluorimétrie en flux (CMF)**

1. Principe

Les cellules cultivées en boîtes de Pétri de diamètre 60 mm ayant atteint la confluence sont mises en présence des produits selon l'invention comportant au moins un ligand osidique. Par exemple, selon un mode de réalisation particulièrement avantageux de l'invention, ces produits selon l'invention porteurs de ligands osidiques sont incorporés à la phase lipidique des liposomes et se retrouvent à la surface des liposomes avec les ligands osidiques librement accessibles de l'extérieur.

Pour la mise en oeuvre de cette étude, il est nécessaire d'encapsuler dans les liposomes une substance fluorescente. De préférence, on utilise le sel potassique de la 5-(6)-carboxyfluorescéine [5-(6)-CF$\gamma$.

Selon cette technique, on compare l'endocytose des différents types de liposomes par mesure de la 5-(6)-CF incorporée dans chaque cellule.

Pour cette technique, il faut éviter d'utiliser une trop forte concentration en 5-(6)-CF qui pourrait induire une extinction de fluorescence.

2. Composition des liposomes préparés comme décrit à l'exemple 1

Liposome "nu" (sans sucre) T

| | |
|---|---|
| Composition | : DPPC / DCP / chol |
| | : 5 / 1 / 4 en moles |
| Concentrations | : 100 mg / 10 ml → 1 % |

Liposome PE-Rha (modèle rhamnose) (= exemple 1 de l'invention)

| | |
|---|---|
| Composition | : PE-Rha / DPPC / DCP / chol |
| | : 1 / 4 / 1 / 4 en moles |
| Concentrations | : 100 mg / 10 ml → 1 % |

Liposome asiaticoside (glucose-glucose-rhamnose) (= exemple 3 de l'invention)

| | |
|---|---|
| Composition | : C.A. / DPPC / DCP / chol |
| | 1 / 4 / 1 / 4 en moles |
| Concentrations | : 100 mg / 10 ml → 1 % |

Liposome séricoside (glucose) (= exemple 8 comparatif)

| | |
|---|---|
| Composition | : SER. / DPPC / DCP / chol |
| | 1 / 4 / 1 / 4 en moles |
| Concentrations | : 100 mg / 10 ml → 1 % |

Le composé fluorescent encapsulé

5-(6) CF : 50 mM dans du PBS

3. Méthode

- Chaque suspension liposomale purifiée sur colonne est diluée au 1/2 dans du milieu de culture.
- On répartit dans chaque boîte (60 mm) 2 ml de suspension liposomale.
- Le blanc est réalisé avec du milieu de culture.
- Les boîtes de Pétri sont placées à l'étuve (37 °C) pendant 1 h 30 min.
- Les boîtes sont rincées deux fois avec 5 ml de tampon PBS.
- Les cellules sont décrochées du support plastique pour les fibroblastes : trypsine 0,25 % pour les kératinocytes : trypsine 0,25 % + EDTA 0,02 %
- Les cellules sont récupérées et centrifugées à 1 500 rpm (centrifugeuse JOUAN E 96) à 4 °C.

. Le surnageant est jeté, le culot celluaire remis en suspension dans 50 µl d'iodure de propidium (I.P.) à 50 ug/ml final dans PBS (test viabilité) .
. La suspension est filtrée sur toile à bluter (diamètre pores : 60 µm) pour éliminer les aggrégats.
. Les cellules sont ensuite gardées dans la glace jusqu'à l'analyse.

4. Mesures :

Les mesures sont réalisées à l'aide d'un cytofluorimètre en flux (EPICS Profile Coultronics) en utilisant une longueur d'onde d'excitation $\lambda_{ex}$ de 488 nm. Les longueurs d'onde d'émission ($\lambda_{em}$) sont :

λ em (5-(6)CF) = 520 nm (vert)
λ em (I.P.) = 620 nm (rouge)

. Un premier histogramme biparamétrique, taille cellulaire = f (densité cellulaire) permet de cerner la population cellulaire (A).
. Cette population sera analysée en fluorescence rouge (B). Seules les cellules non fluorescentes (vivantes) seront considérées pour l'analyse en fluorescence verte (C).
. L'analyse des cellules en fluorescence verte permettra d'apprécier l'incorporation des différents liposomes marqués à la 5-(6)CF (C).
(cf. figure 1).

(cf. figure 1 : LES DIFFERENTS HISTOGRAMMES permettant l'analyse de la population cellulaire par C.M.F. selon le protocole proposé.

A : granulosité = t (taille) → isolement de la population à analyser (fenêtre 1).
B : nombre de cellules = f (fluorescence rouge) → test de viabilité. L'analyse ne sera poursuivie que pour les cellules vivantes (fenêtre 2).
C : nombre de cellules = f (fluorescence verte) → analyse de la 5-(6) CF incorporée dans les cellules).

. La fluorescence intrinsèque des cellules (blanc) sera soustraite de chaque échantillon.

A la figure 2, on a représenté les histogrammes monoparamétriques obtenus par cette technique de cytométrie en flux (C.M.F.) mettant en évidence l'internalisation du contenu des liposomes dans les kératinocytes (nombre de cellules = f (5-(6)CF) incorporée ; type cellulaire ; kératinocytes, nombre analysé 7000 cellules, après interaction avec différents liposomes encapsulant de la 5-(6)CF. incubation : 1 h 30).

On obtient le tableau 2 suivant :

Tableau 2

| relatif à la figure 2 | | |
|---|---|---|
| Liposomes | Moyenne de fluorescence (U.A.) | Rapport au témoin Essai/témoin |
| Témoin | 28,3 | 1 |
| SER | 28,9 | 1,0 |
| PE-Rha | 39,1 | 1,4 |
| C.A. | 47,1 | 1,7 |
| U.A. : unité arbitraire | | |

On observe à partir du tableau 2 et de la figure 2 que les liposomes exposant du rhamnose référencés PE-Rha sont endocytés plus rapidement que les liposomes possédant du glucose de la molécule de séricoside référencés SER. D'autre part, l'endocytose des liposomes contenant l'asiaticoside référencés C.A., est plus importante que celle du PE-Rha.

On observe que la structure des molécules d'asiaticoside et de DPPE-Rha étant très différente, le seul point commun qui les rattache est la présence de rhamnose en bout de chaîne.

Ceci démontre que c'est ce sucre (ligand) qui est responsable de l'augmentation de l'incorporation par endocytose des liposomes dans les kératinocytes. On observera en outre que le liposome comportant à sa surface l'asiaticoside (C.A.) est endocyté plus rapidement sans doute grâce à la présence du bras espaceur glucosylglucose qui le rend plus accessible à son récepteur de la membrane des kératinocytes que le PE-Rha.

Relativement à l'incorporation de ces liposomes dans les fibroblastes : les fibroblastes sont cultivés comme précédemment décrit. On obtient les histogrammes monoparamétriques de la figure 3, (nombre de cellules = f (5-(6) CF) incorporées ; type cellulaire : fibroblastes, nombre analysé 5000 cellules, après interaction avec différents liposomes encapsulant de la 5-(6) CF. Incubation 1 h 30) ainsi que le tableau 3 suivant :

Tableau 3

| relatif à la figure 3 - Fibroblastes | | |
|---|---|---|
| Liposomes | Moyenne de fluorescence (U.A.) | Rapport au témoin Essai/témoin |
| Témoin | 34,4 | 1 |
| SER | 37,0 | 1,1 |
| PE-Rha | 22,0 | 0,6 |
| C.A. | 32,6 | 0,9 |
| U.A. : unité arbitraire | | |

On peut observer que vis-à-vis des fibroblastes, aucun liposomes ne semble être endocyté plus facilement que le liposome témoin.

On peut conclure des analyses de fluorescence précédentes que les Kératinocytes endocytent mieux les liposomes ayant dans leur composition membranaire des molécules comportant un ligand rhamnose, galactose ou galactose-6-phosohate . En outre, on observe que l'α-L-rhamnose et l'α-D-galactose-6-phosphate présentent une spécificité pour les récepteurs membranaires des kératinocytes nettement plus importante que l'α-D-galactose.

Par contre, les fibroblastes sont indifférents à ces molécules ainsi qu'à la structure du glucose porté par le séricoside. On doit donc en conclure que des produits selon l'invention comportant au moins un résidu osidique constitué par le rhamnose, le galactose ou le galactose-6-phosphate et plus particulièrement l'α-L-rhamnose et l'α-D-galactose-6-phosphate peuvent être fixés spécifiquement à la surface des membranes des kératinocytes.

On donnera maintenant divers exemples de composition cosmétique ou pharmaceutique, notamment dermatologique, incorporant des procuits selon l'invention.

Exemple 10

Crème cosmétique antirides

La crème est obtenue par mélange d'une émulsion et d'une suspension gélifiée de liposomes de compositions suivantes :

| émulsion : | |
|---|---|
| perhydrosqualène | 39,2 g |
| lécithine de soja | 0,8 g |
| eau distillée qsp | 220,0 g |

| suspension gélifiée de liposomes : | |
|---|---|
| dipalmitoylphosphatidylcholine (DPPC) | 0,49 g |
| cholestérol (chol) | 0,26 g |
| dicétylphosphate (DCP) | 0,09 g |
| DPPE - rhamnosylée (exemple 1) | 0,16 g |
| polypeptides d'élastine | 0,5 g |
| hydrolysat de thymus de veau | 0,1 g |
| Carbopol 940® | 1,0 g |
| triéthanolamine | 1,0 g |
| eau distillée, parfums et conservateurs qsp | 100,0 g |

On chauffe au bain-marie à 70°C pendant 15 min le mélange de perhydrosqualéne (39,2 g) et de lécithine de soja (0,8 g). La phase grasse obtenue est reprise par 180 ml d'eau distillée, puis on émulsionne au moyen d'un agitateur Raynerie.

Par ailleurs, on prépare une suspension gélifiée de liposomes comme suit. Les liposomes sont préparés comme indiqué à l'exemple 1. Les constituants de la phase lipidique (DPPC, chol, DCP, DPPE-rhamnose) sont dissous dans un mélange chloroforme-méthanol 7:1. La solution organique ainsi obtenue est évaporée sous pression réduite, puis le résidu lipidique est repris par 49 g d'une solution aqueuse contenant les substances à encapsuler (polypeptides d'élastine, et hydrolysat de thymus de veau préparé, par exemple selon le procédé décrit dans le document FR-A1-2 594 847). On obtient ainsi, après sonication, une suspension de liposomes homogénéisée. Cette suspension est enfin gélifiée par addition de 50 g de gel de Carbopol 940® à 2 %, neutralisé à la triéthanolamine, préparé de manière classique.

Cette crème est appliquée quotidiennement sur la peau du visage et du cou.

Exemple 11

Lotion pour traiter le psoriasis

| Composition : | |
|---|---|
| dipalmitoylphosphatidylcholine (DPPC) | 2,2 g |
| cholestérol (chol) | 1,16 g |
| dicétylphosphate (DCP) | 0,41 g |
| asiaticoside (C.A.) | 0,73 g |
| théophylline | 1,00 g |
| conservateur | 0,10 g |
| excipient aqueux gélifié au Carbopol 940® (à 0,1 %) | 100,00 g |

On prépare une poudre lipidique selon l'exemple 3 par atomisation d'une solution dans un mélange dichlorométhane-méthanol 8:2 contenant les constituants lipidiques ou hydrophobes (DPPC, chol, DCP, C.A.) et 0,5 g de théophylline.

Cette poudre est ensuite dispersée dans 50 ml d'une solution aqueuse contenant 0,5 g de théophylline, par agitation, suivie d'une homogénéisation aux ultrasons, selon la méthode connue de l'homme de l'art.

On obtient ainsi une suspension de liposomes encapsulant de la théophylline et comportant en surface des résidus rhamnosyles. On mélange enfin cette suspension à environ 45 g de gel de carbomère stabilisé, préparé de manière classique.

La lotion ainsi obtenue peut être utilisée en applications locales pour le traitement du psoriasis, deux fois par jour, jusqu'à disparition des lésions.

Exemple 12

Lotion capillaire

| Composition : | |
|---|---|
| phospholipides de soja (PS) | 2,5 g |
| cholestérol (chol) | 1,2 g |
| dicétylphosphate (DCP) | 0,5 g |
| asiaticoside (C.A.) | 0,8 g |
| extrait placentaire | 5,0 g |
| pantothénate de sodium | 0,1 g |
| conservateur | 0,1 g |
| excipient aqueux gélifié au Carbopol 940® (à 0,1 %) qsp | 100,0 g |

On opère comme à l'exemple 11, si ce n'est que la poudre lipidique ne contient pas de théophylline.

Cette poudre est dispersée dans 50 ml d'une solution aqueuse contenant 5 g d'extrait placentaire et 0,1 g de pantothénate de sodium, puis gélifiée par la quantité suffisante de gel de carbomère stabilisé pour obtenir un poids final de 100 g de lotion.

Appliquée quotidiennement sur le cuir chevelu, cette lotion améliore l'état de santé du bulbe capillaire et stimule la croissance des cheveux.

**Revendications**
**Revendications pour les Etats contractants suivants : DE, GB, FR, IT, NL, CH, LI, BE, LU**

**1.** Procédé autre que procédé de traitement thérapeutique, pour fixer un produit sur la membrane d'un kératinocyte au moyen d'une liaison ligand-récepteur, caractérisé en ce que l'on utilise un produit constitué d'un produit de base couplé à au moins un ligand constitué par un résidu osidique accessible aux récepteurs membranaires, ledit résidu osidique étant choisi parmi le rhamnose, le galactose, et le galactose-6-phosphate, de préférence l'α-L-rhamnose et l'α-D-galactose-6-phosphate.

**2.** Procédé selon la revendication 1, caractérisé en ce que le ligand est couplé à la surface du produit de base par une liaison chimique covalente.

**3.** Procédé selon l'une des revendications 1 ou 2, caractérisé en ce que le ligand est couplé à la surface du produit de base précité par l'intermédiaire d'un bras espaceur tel que le résidu d'un réactif hétérobifonctionnel ou un groupe de plusieurs sucres.

**4.** Procédé selon la revendication 3, caractérisé en ce que le bras espaceur est constitué par un groupe de plusieurs sucres, tel que le groupe (1-4)-O-β-D-glucopyranosyl-(1-6)-O-β-D-glucopyranosyle.

**5.** Procédé selon l'une des revendications 1 à 4, caractérisé en ce que le produit de base précité est une particule submicroscopique telle qu'un liposome ou une nanoparticule polymérique.

**6.** Procédé selon l'une des revendications 1 à 5, caractérisé en ce que le produit ou le produit de base précité est une molécule ou une macromolécule d'origine naturelle ou synthètique.

**7.** Procédé selon la revendication 6, caractérisé en ce que la molécule ou la macromolécule est un asiaticoside, un digalactosyldiglycéride ou une néoglycoprotéine obtenue par exemple en combinant une albumine de sérum avec un sucre précité.

**8.** Procédé selon l'une des revendications 1 à 7, caractérisé en ce que le produit de base est ou contient une substance d'intérêt cosmétique ou pharmaceutique, notamment dermatologique, telle qu'un agent modulateur du métabolisme des cellules de la peau.

**9.** Procédé pour sélectionner un produit destiné à être fixé sur la membrane d'un kératinocyte au moyen d'une liaison ligand-récepteur, caractérisé en ce que l'on choisit parmi un ensemble de produits ceux

constitués d'un produit de base couplé à au moins un ligand constitué par un résidu osidique accessible aux récepteurs membranaires, ledit résidu osidique étant choisi parmi le rhamnose, le galactose, ou le galactose-6-phosphate, de préférence parmi l'α-L-rhamnose et l'α-D-galactose-6-phosphate.

10. Procédé selon la revendication 9, caractérisé en ce que le produit de base précité est une particule submicroscopique, tel qu'un liposome ou une nanoparticule polymérique.

11. Procédé selon l'une des revendications 9 ou 10, caractérisé en ce que le produit ou le produit de base précité est une molécule ou une macromolécule d'origine naturelle ou synthétique.

12. Procédé selon la revendication 11, caractérisé en ce que la molécule naturelle ou synthétique est un asiaticoside, un digalactosyldiglycéride ou une néoglycoprotéine obtenue par exemple en combinant une albumine de sérum avec un sucre précité.

13. Procédé selon l'une des revendications 9 à 12, caractérisé en ce que le produit ou le produit de base est ou contient une substance d'intérêt cosmétique ou pharmaceutique, notamment dermatologique, telle qu'un agent modulateur du métabolisme des cellules de la peau.

14. Procédé de préparation d'un produit destiné à être fixé sur la membrane d'un kératinocyte au moyen d'une liaison ligand-récepteur, caractérisé en ce que l'on couple un produit de base correspondant à au moins un ligand constitué par un résidu osidique accessible aux récepteurs membranaires, ledit résidu osidique étant choisi parmi le rhamnose, le galactose et le galactose-6-phosphate, de préférence l'α-L-rhamnose et l'α-D-galactose-6-phosphate.

15. Procédé selon la revendication 14, caractérisé en ce que le ligand est couplé à la surface du produit de base précité par une liaison chimique covalente.

16. Procédé selon la revendication 14 ou 15, caractérisé en ce que le ligand précité est couplé au produit de base par l'intermédiaire d'un bras espaceur tel que le résidu d'un réactif hétérobifonctionnel ou un groupe de plusieurs sucres.

17. Procédé selon la revendication 16, caractérisé en ce que le bras espaceur est constitué par un groupe de plusieurs sucres, tel que le groupe (1-4)-O-β-D-glucopyranosyl-(1-6)-O-β-D-glucopyranosyle.

18. Procédé selon l'une des revendications 14 à 17, caractérisé en ce qu'on utilise comme produit de base une particule submicroscopique telle qu'un liposome ou une nanoparticule polymérique.

19. Procédé selon la revendication 18, caractérisé en ce que la proportion en pourcentage molaire des résidus osidiques par rapport à l'ensemble des molécules lipidiques formant la bicouche du liposome est d'au moins 10 %, et de préférence d'au moins 15 % par rapport à l'ensemble des lipides amphiphiles.

20. Procédé selon l'une quelconque des revendications 18 ou 19, caractérisé en ce que les liposomes sont formulés de manière à présenter une forte microviscosité.

21. Procédé selon l'une des revendications 14 à 17, caractérisé en ce qu'on utilise comme produit de base une molécule ou une macromolécule d'origine naturelle ou synthétique, telle qu'une protéine, en particulier une albumine de sérum.

22. Procédé selon l'une des revendications 14 à 21, caractérisé en ce que le produit ou le produit de base précité est une substance d'intérêt cosmétique ou pharmaceutique, notamment dermatologique, telle qu'un agent modulateur du métabolisme des cellules de la peau.

23. Procédé selon l'une des revendications 14 à 21, caractérisé en ce que le produit de base ou le produit précité contient une substance d'intérêt cosmétique ou pharmaceutique, notamment dermatologique, telle qu'un agent modulateur du métabolisme des cellules de la peau.

24. Produit destiné à être fixé sur la membrane d'un kératinocyte au moyen d'une liaison ligand-récepteur, caractérisé en ce qu'il est susceptible d'être obtenu selon un procédé défini à l'une quelconque des revendications 9 à 23.

25. Produit destiné à être fixé sur la membrane d'un kératinocyte au moyen d'un liaison ligand-récepteur, caractérisé en ce qu'il comprend au moins un ligand constitué par un résidu osidique accessible aux récepteurs membranaires, ledit résidu osidique étant choisi parmi le rhamnose, le galactose, et le galactose-6-phosphate, de préférence l'α-L-rhamnose et l'α-D-galactose-6-phosphate, couplé à un produit de base choisi notamment parmi le groupe constitué par une particule submicroscopique telle qu'un liposome ou une nanoparticule polymérique ; une molécule ou macromolécule d'origine naturelle ou synthétique, telle qu'une protéine, en particulier de l'albumine de sérum ; une substance d'intérêt cosmétique ou pharmaceutique, notamment dermatologique, telle qu'un agent modulateur du métabolisme des cellules de la peau.

26. Produit selon la revendication 25, caractérisé en ce que le couplage précité est réalisé par l'intermédiaire d'un bras espaceur, tel que le résidu d'un réactif hétérobifonctionnel ou un groupe de plusieurs sucres, tel le groupe (1-4)-O-β-D-glucopyranosyl-(1-6)-O-β-D-glucopyranosyle.

27. Produit selon la revendication 25 ou 26, caractérisé en ce que le produit de base est une protéine et le nombre de résidus osidiques précités formant ligand est d'au moins 20 environ.

28. Produit selon l'une des revendications 25 à 27, caractérisé en ce que la nanoparticule polymérique précitée porte en surface des résidus α-D-galactosyle, α-L-rhamnosyle ou α-D-galactopyranosyle-6-phosphate.

29. Produit selon l'une des revendications 25 à 27, caractérisé en ce que le liposome précité porte en surface des résidus α-L-rhamnosyle ou α-D-galactosyle-6-phosphate.

30. Produit selon l'une des revendications 25 à 26, caractérisé en ce qu'il est choisi parmi le groupe constitué par un asiaticoside en particulier extrait de la plante Centella asiatica ; un digalactosyldiglycéride ; une néoglycoprotéine, en particulier obtenue par la combinaison d'albumine de sérum avec l'α-L-rhamnose ou l'α-D-galactose-6-phosphate.

31. Composition cosmétique ou pharmaceutique, notamment dermatologique, caractérisée en ce qu'elle contient au moins un produit tel qu'obtenu par le procédé selon l'une quelconque des revendications 9 à 23 ou tel que défini par l'une quelconque des revendications 24 à 30.

32. Composition selon la revendication 31, destinée à tout soin ou tout traitement intéressant les kératinocytes, en particulier à l'amélioration de la régénération cutanée, au traitement du psoriasis, à la repousse des cheveux.

33. Procédé de préparation d'une composition cosmétique ou pharmaceutique, notamment dermatologique, caractérisé en ce qu'on associe à un excipient, véhicule ou support cosmétiquement ou pharmaceutiquement acceptable, au moins un produit tel qu'obtenu par le procédé selon l'une quelconque des revendications 9 à 23 ou tel que défini selon l'une quelconque des revendications 24 à 30.

**Revendications pour l'Etat contractant suivant : ES**

1. Procédé autre que procédé de traitement thérapeutique, pour fixer un produit sur La membrane d'un kératinocyte au moyen d'une liaison ligand-récepteur, caractérisé en ce que l'on utilise un produit constitué d'un produit de base couplé à au moins un ligand constitué par un résidu osidique accessible aux récepteurs membranaires, ledit résidu osidique étant choisi parmi le rhamnose, le galactose, et le galactose-6-phosphate, de préférence l'α-L-rhamnose et l'α-D-galactose-6-phosphate.

2. Procédé selon La revendication 1, caractérisé en ce que le ligand est couplé à la surface du produit de base par une liaison chimique covalente.

3. Procédé selon l'une des revendications 1 ou 2, caractérisé en ce que le ligand est couplé à la surface du produit de base précité par l'intermédiaire d'un bras espaceur tel que le résidu d'un réactif hétérobifonctionnel ou un groupe de plusieurs sucres.

4. Procédé selon la revendication 3, caractérisé en ce que le bras espaceur est constitué par un groupe de plusieurs sucres, tel que le groupe (1-4)-O-$\beta$-D-glucopyranosyl-(1-6)-O-$\beta$-D-glucopyranosyle.

5. Procédé selon l'une des revendications 1 à 4, caractérisé en ce que le produit de base précité est une particule submicroscopique telle qu'un liposome ou une nanoparticule polymérique.

6. Procédé selon l'une des revendications 1 à 5, caractérisé en ce que le produit ou le produit de base précité est une molécule ou une macromolécule d'origine naturelle ou synthétique.

7. Procédé selon la revendication 6, caractérisé en ce que la molécule ou la macromolécule est un asiaticoside, un digalactosyldiglycéride ou une néoglycoprotéine obtenue par exemple en combinant une albumine de sérum avec un sucre précité.

8. Procédé selon l'une des revendications 1 à 7, caractérisé en ce que le produit ou le produit de base est ou contient une substance d'intérêt cosmétique ou pharmaceutique, notamment dermatologique, telle qu'un agent modulateur du métabolisme des cellules de la peau.

9. Procédé pour sélectionner un produit destiné à être fixé sur la membrane d'un kératinocyte au moyen d'une liaison ligand-récepteur, caractérisé en ce que l'on choisit parmi un ensemble de produits ceux constitués d'un produit de base couplé à au moins un ligand constitué par un résidu osidique accessible aux récepteurs membranaires, ledit résidu osidique étant choisi parmi le rhamnose, le galactose, ou le galactose-6-phosphate, de préférence parmi l'$\alpha$-L-rhamnose et l'$\alpha$-D-galactose-6-phosphate.

10. Procédé selon la revendication 9, caractérisé en ce que le produit de base précité est une particule submicroscopique, tel qu'un liposome ou une nanoparticule polymérique.

11. Procédé selon l'une des revendications 9 ou 10, caractérisé en ce que le produit ou le produit de base précité est une molécule ou une macromolécule d'origine naturelle ou synthétique.

12. Procédé selon la revendication 11, caractérisé en ce que la molécule naturelle ou synthétique est un asiaticoside, un digalactosyldiglycéride ou une néoglycoprotéine obtenue par exemple en combinant une albumine de sèrum avec un sucre précité.

13. Procédé selon l'une des revendications 9 à 12, caractérisé en ce que le produit ou le produit de base est ou contient une substance d'intérêt cosmétique ou pharmaceutique, notamment dermatologique, telle qu'un agent modulateur du métabolisme des cellules de la peau.

14. Procédé de préparation d'un produit destiné à être fixé sur la membrane d'un kératinocyte au moyen d'une liaison ligand-récepteur, caractérisé en ce que l'on couple un produit de base correspondant à au moins un ligand constitué par un résidu osidique accessible aux récepteurs membranaires, ledit résidu osidique étant choisi parmi le rhamnose, le galactose et le galactose-6-phosphate, de préférence l'$\alpha$-L-rhamnose et l'$\alpha$-D-galactose-6-phosphate.

15. Procédé selon la revendication 14, caractérisé en ce que le ligand est couplé à la surface du produit de base précité par une liaison chimique covalente.

16. Procédé selon la revendication 14 ou 15, caractérisé en ce que le ligand précité est couplé au produit de base par l'intermédiaire d'un bras espaceur tel que le résidu d'un réactif hétérobifonctionnel ou un groupe de plusieurs sucres.

17. Procédé selon la revendication 16, caractérisé en ce que le bras espaceur est constitué par un groupe de plusieurs sucres, tel que le groupe (1-4)-O-$\beta$-D-glucopyranosyl-(1-6)-O-$\beta$-D-glucopyranosyle.

**18.** Procédé selon l'une des revendications 14 à 17, caractérisé en ce qu'on utilise comme produit de base une particule submicroscopique telle qu'un liposome ou une nanoparticule polymérique.

**19.** Procédé selon la revendication 18, caractérisé en ce que la proportion en pourcentage molaire des résidus osidiques par rapport à l'ensemble des molécules lipidiques formant la bicouche du liposome est d'au moins 10 %, et de préférence d'au moins 15 % par rapport à l'ensemble des lipides amphiphiles.

**20.** Procédé selon l'une quelconque des revendications 18 ou 19, caractérisé en ce que les liposomes sont formulés de manière à présenter une forte microviscosité.

**21.** Procédé selon l'une des revendications 14 à 17, caractérisé en ce qu'on utilise comme produit de base une molécule ou une macromolécule d'origine naturelle ou synthétique, telle qu'une protéine, en particulier une albumine de sérum.

**22.** Procédé selon l'une des revendications 14 à 21, caractérisé en ce que le produit ou le produit de base précité est une substance d'intérêt cosmétique ou pharmaceutique, notamment dermatologique, telle qu'un agent modulateur du métabolisme des cellules de la peau.

**23.** Procédé selon l'un des revendications 14 à 21, caractérisé en ce que le produit ou le produit de base précité contient une substance d'intérêt cosmétique ou pharmaceutique, notamment dermatologique, telle qu'un agent modulateur du métabolisme des cellules de la peau.

**24.** Procédé de préparation d'une composition cosmétique ou pharmaceutique, notamment dermatologique, caractérisé en ce qu'on associe à un excipient, véhicule ou support cosmétiquement ou pharmaceutiquement acceptable, au moins un produit tel qu'obtenu par le procédé selon l'une quelconque des revendications 9 à 23.

**25.** Procédé selon la revendication 24, caractérisé en ce que la composition est destinée à tout soin ou tout traitement intéressant les kératinocytes, en particulier à l'amélioration de la régénération cutanée, au traitement du psoriasis, à la repousse des cheveux.

**Claims**

**Claims for the following Contracting States : DE, GB, FR, IT, NL, CH, LI, BE, LU**

**1.** Method, other than a method of therapeutic treatment, of binding a product to the membrane of a keratinocyte by means of a ligand-receptor bond, characterized in that a product consisting of a basic product coupled to at least one ligand consisting of an oside residue accessible to the membrane receptors is used, said oside residue being selected from rhamnose, galactose and galactose-6-phosphate, preferably α-L-rhamnose and α-D-galactose-6-phosphate.

**2.** Method according to claim 1, characterized in that the ligand is coupled to the surface of the basic product by a covalent chemical bond.

**3.** Method according to one of claims 1 or 2, characterized in that the ligand is coupled to the surface of the afore-mentioned basic product via a spacer arm such as the residue of a heterobifunctional reagent or a group of several sugars.

**4.** Method according to claim 3, characterized in that the spacer arm consists of a group of several sugars like the (1-4)-O-β-D-glucopyranosyl-(1-6)-O-β-D-glucopyranosyl group.

**5.** Method according to one of claims 1 to 4, characterized in that the afore-mentioned basic product is a submicroscopic particle such as a liposome or a polymeric nanoparticle.

**6.** Method according to one of claims 1 to 5, characterized in that the afore-mentioned product or basic product is a molecule or a macromolecule of natural or synthetic origin.

7. Method according to claim 6, characterized in that the molecule or macromolecule is an asiaticoside, a digalactosyldiglyceride or a neoglycoprotein obtained for example by combining a serum albumin with an afore-mentioned sugar.

8. Method according to one of claims 1 to 7, characterized in that the basic product is or contains a substance of cosmetic or pharmaceutical interest, especially dermatological interest, such as an agent for modulating the metabolism of the skin cells.

9. Method of selecting a product which is to be bound to the membrane of a keratinocyte by means of a ligand-receptor bond, characterized in that, from a group of products, those consisting of a basic product coupled to at least one ligand consisting of an oside residue accessible to the membrane receptors are selected, said oside residue being selected from rhamnose, galactose or galactose-6-phosphate, preferably from α-L-rhamnose and α-D-galactose-6-phosphate.

10. Method according to claim 9, characterized in that the afore-mentioned basic product is a submicroscopic particle such as a liposome or a polymeric nanoparticle.

11. Method according to one of claims 9 or 10, characterized in that the afore-mentioned product or basic product is a molecule or a macromolecule of natural or synthetic origin.

12. Method according to claim 11, characterized in that the natural or synthetic molecule is an asiaticoside, a digalactosyldiglyceride or a neoglycoprotein obtained for example by combining a serum albumin with an afore-mentioned sugar.

13. Method according to one of claims 9 to 12, characterized in that the product or basic product is or contains a substance of cosmetic or pharmaceutical interest, especially dermatological interest, such as an agent for modulating the metabolism of the skin cells.

14. Method of preparing a product which is to be bound to the membrane of a keratinocyte by means of a ligand-receptor bond, characterized in that a corresponding basic product is coupled to at least one ligand consisting of an oside residue accessible to the membrane receptors, said oside residue being selected from rhamnose, galactose and galactose-6-phosphate, preferably α-L-rhamnose and α-D-galactose-6-phosphate.

15. Method according to claim 14, characterized in that the ligand is coupled to the surface of the afore-mentioned basic product by a covalent chemical bond.

16. Method according to claim 14 or 15, characterized in that the afore-mentioned ligand is coupled to the basic product via a spacer arm such as the residue of a heterobifunctional reagent or a group of several sugars.

17. Method according to claim 16, characterized in that the spacer arm consists of a group of several sugars like the (1-4)-O-β-D-glucopyranosyl-(1-6)-O-β-D-glucopyranosyl group.

18. Method according to one of claims 14 to 17, characterized in that the basic product used is a submicroscopic particle such as a liposome or a polymeric nanoparticle.

19. Method according to claim 18, characterized in that the proportion of oside residues, as a molar percentage of all the lipid molecules forming the bilayer of the liposome, is at least 10% and preferably at least 15% relative to all the amphiphilic lipids.

20. Method according to any one of claims 18 or 19, characterized in that the liposomes are formulated so as to have a high microviscosity.

21. Method according to one of claims 14 to 17, characterized in that the basic product used is a molecule or macromolecule of natural or synthetic origin, such as a protein, in particular a serum albumin.

22. Method according to one of claims 14 to 21, characterized in that the afore-mentioned product or basic product is a substance of cosmetic or pharmaceutical interest, especially dermatological interest, such as an agent for modulating the metabolism of the skin cells.

23. Method according to one of claims 14 to 21, characterized in that the afore-mentioned basic product or product contains a substance of cosmetic or pharmaceutical interest, especially dermatological interest, such as an agent for modulating the metabolism of the skin cells.

24. Product which is to be bound to the membrane of a keratinocyte by means of a ligand-receptor bond, characterized in that it is capable of being obtained by a method defined in any one of claims 9 to 23.

25. Product which is to be bound to the membrane of a keratinocyte by means of a ligand-receptor bond, characterized in that it comprises at least one ligand consisting of an oside residue accessible to the membrane receptors, said oside residue being selected from rhamnose, galactose and galactose-6-phosphate, preferably α-L-rhamnose and α-D-galactose-6-phosphate, and coupled to a basic product selected especially from the group consisting of a submicroscopic particle such as a liposome or a polymeric nanoparticle; a molecule or macromolecule of natural or synthetic origin, such as a protein, in particular serum albumin; and a substance of cosmetic or pharmaceutical interest, especially dermatological interest, such as an agent for modulating the metabolism of the skin cells.

26. Product according to claim 25, characterized in that the afore-mentioned coupling is effected via a spacer arm such as the residue of a heterobifunctional reagent or a group of several sugars like the (1-4)-O-β-D-glucopyranosyl-(1-6)-O-β-D-glucopyranosyl group.

27. Product according to claim 25 or 26, characterized in that the basic product is a protein and the number of afore-mentioned oside residues forming the ligand is at least about 20.

28. Product according to one of claims 25 to 27, characterized in that the afore-mentioned polymeric nanoparticle carries α-D-galactosyl, α-L-rhamnosyl or α-D-galactopranosyl-6-phosphate residues on the surface.

29. Product according to one of claims 25 to 27, characterized in that the afore-mentioned liposome carries α-L-rhamnosyl or α-D-galactosyl-6-phosphate residues on the surface.

30. Product according to one of claims 25 to 26, characterized in that it is selected from the group consisting of an asiaticoside extracted in particular from the plant Centella asiatica; a digalactosyldiglyceride; and a neoglycoprotein obtained in particular by combining serum albumin with α-L-rhamnose or α-D-galactose-6-phosphate.

31. Cosmetic or pharmaceutical composition, especially dermatological composition, characterized in that it contains at least one product such as obtained by the method according to any one of claims 9 to 23 or such as defined by any one of claims 24 to 30.

32. Composition according to claim 31 which is intended for any care or any treatment affecting the keratinocytes, in particular for the improvement of skin regeneration, the treatment of psoriasis or renewed hair growth.

33. Method of preparing a cosmetic or pharmaceutical composition, especially a dermatological composition, characterized in that at least one product such as obtained by the method according to any one of claims 9 to 23, or such as defined according to any one of claims 24 to 30, is associated with a cosmetically or pharmaceutically acceptable excipient, vehicle or carrier.

**Claims for the following Contracting State : ES**

1. Method, other than a method of therapeutic treatment, of binding a product to the membrane of a keratinocyte by means of a ligand-receptor bond, characterized in that a product consisting of a basic product coupled to at least one ligand consisting of an oside residue accessible to the membrane receptors is used, said oside residue being selected from rhamnose, galactose and galactose-6-

phosphate, preferably α-L-rhamnose and α-D-galactose-6-phosphate.

**2.** Method according to claim 1, characterized in that the ligand is coupled to the surface of the basic product by a covalent chemical bond.

**3.** Method according to one of claims 1 or 2, characterized in that the ligand is coupled to the surface of the afore-mentioned basic product via a spacer arm such as the residue of a heterobifunctional reagent or a group of several sugars.

**4.** Method according to claim 3, characterized in that the spacer arm consists of a group of several sugars like the (1-4)-O-β-D-glucopyranosyl-(1-6)-O-β-D-glucopyranosyl group.

**5.** Method according to one of claims 1 to 4, characterized in that the afore-mentioned basic product is a submicroscopic particle such as a liposome or a polymeric nanoparticle.

**6.** Method according to one of claims 1 to 5, characterized in that the afore-mentioned product or basic product is a molecule or a macromolecule of natural or synthetic origin.

**7.** Method according to claim 6, characterized in that the molecule or macromolecule is an asiaticoside, a digalactosyldiglyceride or a neoglycoprotein obtained for example by combining a serum albumin with an afore-mentioned sugar.

**8.** Method according to one of claims 1 to 7, characterized in that the product or basic product is or contains a substance of cosmetic or pharmaceutical interest, especially dermatological interest, such as an agent for modulating the metabolism of the skin cells.

**9.** Method of selecting a product which is to be bound to the membrane of a keratinocyte by means of a ligand-receptor bond, characterized in that, from a group of products, those consisting of a basic product coupled to at least one ligand consisting of an oside residue accessible to the membrane receptors are selected, said oside residue being selected from rhamnose, galactose or galactose-6-phosphate, preferably from α-L-rhamnose and α-D-galactose-6-phosphate.

**10.** Method according to claim 9, characterized in that the afore-mentioned basic product is a submicroscopic particle such as a liposome or a polymeric nanoparticle.

**11.** Method according to one of claims 9 or 10, characterized in that the afore-mentioned product or basic product is a molecule or a macromolecule of natural or synthetic origin.

**12.** Method according to claim 11, characterized in that the natural or synthetic molecule is an asiaticoside, a digalactosyldiglyceride or a neoglycoprotein obtained for example by combining a serum albumin with an afore-mentioned sugar.

**13.** Method according to one of claims 9 to 12, characterized in that the product or basic product is or contains a substance of cosmetic or pharmaceutical interest, especially dermatological interest, such as an agent for modulating the metabolism of the skin cells.

**14.** Method of preparing a product which is to be bound to the membrane of a keratinocyte by means of a ligand-receptor bond, characterized in that a corresponding basic product is coupled to at least one ligand consisting of an oside residue accessible to the membrane receptors, said oside residue being selected from rhamnose, galactose and galactose-6-phosphate, preferably α-L-rhamnose and α-D-galactose-6-phosphate.

**15.** Method according to claim 14, characterized in that the ligand is coupled to the surface of the afore-mentioned basic product by a covalent chemical bond.

**16.** Method according to claim 14 or 15, characterized in that the afore-mentioned ligand is coupled to the basic product via a spacer arm such as the residue of a heterobifunctional reagent or a group of several sugars.

17. Method according to claim 16, characterized in that the spacer arm consists of a group of several sugars like the (1-4)-O-β-D-glucopyranosyl-(1-6)-O-β-D-glucopyranosyl group.

18. Method according to one of claims 14 to 17, characterized in that the basic product used is a submicroscopic particle such as a liposome or a polymeric nanoparticle.

19. Method according to claim 18, characterized in that the proportion of oside residues, as a molar percentage of all the lipid molecules forming the bilayer of the liposome, is at least 10% and preferably at least 15% relative to all the amphiphilic lipids.

20. Method according to any one of claims 18 or 19, characterized in that the liposomes are formulated so as to have a high microviscosity.

21. Method according to one of claims 14 to 17, characterized in that the basic product used is a molecule or macromolecule of natural or synthetic origin, such as a protein, in particular a serum albumin.

22. Method according to one of claims 14 to 21, characterized in that the afore-mentioned product or basic product is a substance of cosmetic or pharmaceutical interest, especially dermatological interest, such as an agent for modulating the metabolism of the skin cells.

23. Method according to one of claims 14 to 21, characterized in that the afore-mentioned product or basic product contains a substance of cosmetic or pharmaceutical interest, especially dermatological interest, such as an agent for modulating the metabolism of the skin cells.

24. Method of preparing a cosmetic or pharmaceutical composition, especially a dermatological composition, characterized in that at least one product such as obtained by the method according to any one of claims 9 to 23, is associated with a cosmetically or pharmaceutically acceptable excipient, vehicle or carrier.

25. Method according to claim 24, characterized in that the composition is intended for any care or any treatment affecting the keratinocytes, in particular for the improvement of skin regeneration, the treatment of psoriasis or renewed hair growth.

**Patentansprüche**

**Patentansprüche für folgende Vertragsstaaten : DE, GB, FR, IT, NL, CH, LI, BE, LU**

1. Verfahren, ausgenommen therapeutisches Behandlungsverfahren, zum Fixieren eines Produkts an der Membran eines Keratinozyts mittels einer Ligand-Rezeptor-Bindung, dadurch gekennzeichnet, daß man ein Produkt verwendet, bestehend aus einem Basisprodukt, das an mindestens einen durch einen für die Membranrezeptoren zugänglichen Glycosidrest gebildeten Ligand gekoppelt ist, wobei der Glycosidrest ausgewählt ist aus Rhamnose, Galactose und Galactose-6-phosphat, vorzugsweise α-L-Rhamnose und α-D-Galactose-6-phosphat.

2. Verfahren nach Anspruch 1, dadurch gekennzeichnet, daß der Ligand durch eine kovalente chemische Bindung an die Oberfläche des Basisprodukts gekoppelt wird.

3. Verfahren nach einem der Ansprüche 1 oder 2, dadurch gekennzeichnet, daß der Ligand unter Zwischenschaltung eines Raumarmes, wie des Restes eines heterobifunktionellen Reagens oder einer Gruppe von mehreren Zuckern, an die Oberfläche des Basisprodukts gekoppelt wird.

4. Verfahren nach Anspruch 3, dadurch gekennzeichnet, daß der Raumarm durch eine Gruppe von mehreren Zuckern, wie die Gruppe (1-4)-O-β-D-Glucopyranosyl-(1-6)-O-β-D-glucopyranosyl, gebildet wird.

5. Verfahren nach einem der Ansprüche 1 bis 4, dadurch gekennzeichnet, daß das Basisprodukt ein submikroskopisches Teilchen, wie ein Liposom oder ein Polymernanoteilchen, ist.

6. Verfahren nach einem der Ansprüche 1 bis 5, dadurch gekennzeichnet, daß das Produkt oder das Basisprodukt ein Molekül oder ein Makromolekül natürlichen oder synthetischen Ursprungs ist.

7. Verfahren nach Anspruch 6, dadurch gekennzeichnet, daß das Molekül oder das Makromolekül ein Asiatikosid, ein Digalactosyldiglycerid oder ein Neoglycoprotein ist, welches beispielsweise durch Kombination eines Serumalbumins mit einem genannten Zucker erhalten wird.

8. Verfahren nach einem der Ansprüche 1 bis 7, dadurch gekennzeichnet, daß das Basisprodukt eine Substanz von kosmetischem oder pharmazeutischem, insbesondere dermatologischem, Interesse ist oder enthält, wie ein(en) Hautzellenstoffwechselmodulator.

9. Verfahren zum Selektieren eines Produkts, das dazu bestimmt ist, an der Membran eines Keratinozyts mittels einer Ligand-Rezeptor-Bindung fixiert zu werden, dadurch gekennzeichnet, daß man aus einer Gruppe von Produkten jene auswählt, die durch ein Rasisprodukt gebildet sind, das an mindestens einen durch einen für die Membranrezeptoren zugänglichen Glycosidrest gebildeten Ligand gekoppelt ist, wobei der Glycosidrest ausgewählt ist aus Rhamnose, Galactose oder Galactose-6-phosphat, vorzugsweise aus α-L-Rhamnose und α-D-Galactose-6-phosphat.

10. Verfahren nach Anspruch 9, dadurch gekennzeichnet, daß das Basisprodukt ein submikroskopisches Teilchen, wie ein Liposom oder ein Polymernanoteilchen, ist.

11. Verfahren nach einem der Ansprüche 9 oder 10, dadurch gekennzeichnet, daß das Produkt oder das Basisprodukt ein Molekül oder ein Makromolekül natürlichen oder synthetischen Ursprungs ist.

12. Verfahren nach Anspruch 11, dadurch gekennzeichnet, daß das natürliche oder synthetische Molekül ein Asiatikosid, ein Digalactosyldiglycerid oder ein Neoglycoprotein ist, welches beispielsweise durch Kombination eines Serumalbumins mit einem genannten Zucker erhalten wird.

13. Verfahren nach einem der Ansprüche 9 bis 12, dadurch gekennzeichnet, daß das Produkt oder das Basisprodukt eine Substanz von kosmetischem oder pharmazeutischem, insbesondere dermatologischem, Interesse ist oder enthält, wie ein(en) Hautzellen-Stoffwechselmodulator.

14. Verfahren zur Herstellung eines Produkts, das dazu bestimmt ist, an der Membran eines Keratinozyts mittels einer Ligand-Rezeptor-Bindung fixiert zu werden, dadurch gekennzeichnet, daß man ein entsprechendes Basisprodukt an mindestens einen durch einen für die Membranrezeptoren zugänglichen Glycosidrest gebildeten Ligand koppelt, wobei der Glycosidrest ausgewählt ist aus Rhamnose, Galactose und Galactose-6-phosphat, vorzugsweise α-L-Rhamnose und α-D-Galactose-6-phosphat.

15. Verfahren nach Anspruch 14, dadurch gekennzeichnet, daß der Ligand durch eine kovalente chemische Bindung an die Oberfläche des Basisprodukts gekoppelt wird.

16. Verfahren nach Anspruch 14 oder 15, dadurch gekennzeichnet, daß der Ligand unter Zwischenschaltung eines Raumarmes, wie des Restes eines heterobifunktionellen Reagens oder einer Gruppe von mehreren Zuckern, an das Basisprodukt gekoppelt wird.

17. Verfahren nach Anspruch 16, dadurch gekennzeichnet, daß der Raumarm durch eine Gruppe von mehreren Zuckern, wie die Gruppe (1-4)-O-β-D-Glucopyranosyl-(1-6)-O-β-D-glucopyranosyl, gebildet wird.

18. Verfahren nach einem der Ansprüche 14 bis 17, dadurch gekennzeichnet, daß man als Basisprodukt ein submikroskopisches Teilchen, wie ein Liposom oder ein Polymernanoteilchen, verwendet.

19. Verfahren nach Anspruch 18, dadurch gekennzeichnet, daß das prozentuelle Molverhältnis der Glycosidreste zur Gesamtheit der die Doppelschicht des Liposoms bildenden Lipidmoleküle mindestens 10 %, vorzugsweise mindestens 15 %, bezogen auf die Gesamtheit der amphiphilen Lipide, ist.

20. Verfahren nach einem der Ansprüche 18 oder 19, dadurch gekennzeichnet, daß die Liposome derart formuliert werden, daß sie eine starke Mikroviskosität aufweisen.

**21.** Verfahren nach einem der Ansprüche 14 bis 17, dadurch gekennzeichnet, daß man als Basisprodukt ein Molekül oder ein Makromolekül natürlichen oder synthetischen Ursprungs, wie ein Protein, insbesondere ein Serumalbumin, verwendet.

**22.** Verfahren nach einem der Ansprüche 14 bis 21, dadurch gekennzeichnet, daß das Produkt oder das Basisprodukt eine Substanz von kosmetischem oder pharmazeutischem, insbesondere dermatologischem, Interesse, wie ein Hautzellenstoffwechselmodulator ist.

**23.** Verfahren nach einem der Ansprüche 14 bis 21, dadurch gekennzeichnet, daß das Produkt oder das Basisprodukt eine Substanz von kosmetischem oder pharmazeutischem, insbesondere dermatologischem Interesse enthält, wie einen Hautzellenstoffwechselmodulator.

**24.** Produkt, das dazu bestimmt ist, an der Membran eines Keratinozyts mittels einer Ligand-Rezeptor-Bindung fixiert zu werden, dadurch gekennzeichnet, daß es nach einem in einem der Ansprüche 9 bis 23 definierten Verfahren hergestellt werden kann.

**25.** Produkt, das dazu bestimmt ist, an der Membran eines Keratinozyts mittels einer Ligand-Rezeptor-Bindung fixiert zu werden, dadurch gekennzeichnet, daß es mindestens einen Ligand umfaßt, der durch einen für die Membranrezeptoren zugänglichen Glycosidrest gebildet ist, wobei der Glycosidrest ausgewählt ist aus Rhamnose, Galactose und Galactose-6-phosphat, vorzugsweise α-L-Rhamnose und α-D-Galactose-6-phosphat, und an ein Basisprodukt gekoppelt ist, welches insbesondere ausgewählt ist aus der Gruppe bestehend aus einem submikroskopischen Teilchen, wie einem Liposom oder einem Polymernanoteilchen; einem Molekül oder Makromolekül natürlichen oder synthetischen Ursprungs, wie einem Protein, insbesondere Serumalbumin; einer Substanz von kosmetischem oder pharmazeutischem, insbesondere dermatologischem, Interesse, wie einem Hautzellenstoffwechselmodulator.

**26.** Produkt nach Anspruch 25, dadurch gekennzeichnet, daß die Kopplung unter Zwischenschaltung eines Raumarmes, wie des Restes eines heterobifunktionellen Reagens oder einer Gruppe von mehreren Zuckern, wie der Gruppe (1-4)-O-β-D-Glucopyranosyl-(1-6)-O-β-D-glucopyranosyl, erfolgt.

**27.** Produkt nach Anspruch 25 oder 26, dadurch gekennzeichnet, daß das Basisprodukt ein Protein ist und die Anzahl an ligandbildenden Glycosidresten mindestens etwa 20 beträgt.

**28.** Produkt nach einem der Ansprüche 25 bis 27, dadurch gekennzeichnet, daß das Polymernanoteilchen an der Oberfläche α-D-Galactosyl-, α-L-Rhamnosyl- oder α-D-Galactopyranosyl-6-phosphat-Reste trägt.

**29.** Produkt nach einem der Ansprüche 25 bis 27, dadurch gekennzeichnet, daß das Liposom an der Oberfläche α-L-Rhamnosyl-oder α-D-Galactosyl-6-phosphat-Reste trägt.

**30.** Produkt nach einem der Ansprüche 25 bis 26, dadurch gekennzeichnet, daß es ausgewählt ist aus der Gruppe bestehend aus einem Asiaticosid, insbesondere einem Extrakt der Pflanze Centella asiatica; einem Digalactosyldiglycerid; einem Neoglycoprotein, insbesondere einem durch die Vereinigung von Serumalbumin mit α-L-Rhamnose oder α-D-Galactose-6-phosphat erhaltenen Neoglycoprotein.

**31.** Kosmetische oder pharmazeutische, insbesondere dermatologische, Zusammensetzung, dadurch gekennzeichnet, daß sie mindestens ein Produkt, wie das durch das Verfahren nach einem der Ansprüche 9 bis 23 erhaltene oder wie das in einem der Ansprüche 24 bis 30 definierte enthält.

**32.** Zusammensetzung nach Anspruch 31 für jede Art von Pflege oder Behandlung, die für Keratinozyten von Interesse ist, insbesondere zur Verbesserung der Hautregeneration, zur Behandlung von Psoriasis und für den Haarnachwuchs.

**33.** Verfahren zur Herstellung einer kosmetischen oder pharmazeutischen, insbesondere dermatologischen, Zusammensetzung, dadurch gekennzeichnet, daß ein kosmetisch oder pharmazeutisch akzeptabler Exzipient, Vehikel oder Träger mit mindestens einem Produkt, wie dem durch das Verfahren nach einem der Ansprüche 9 bis 23 erhaltenen oder wie dem in einem der Ansprüche 24 bis 30 definierten vereinigt wird.

**Patentansprüche für folgenden Vertragsstaat : ES**

1. Verfahren, ausgenommen therapeutisches Behandlungsverfahren, zum Fixieren eines Produkts an der Membran eines Keratinozyts mittels einer Ligand-Rezeptor-Bindung, dadurch gekennzeichnet, daß man ein Produkt verwendet, bestehend aus einem Basisprodukt, das an mindestens einen durch einen für die Membranrezeptoren zugänglichen Osidrest gebildeten Ligand gekoppelt ist, wobei der Osidrest ausgewählt ist aus Rhamnose, Galactose und Galactose-6-phosphat, vorzugsweise α-L-Rhamnose und α-D-Galactose-6-phosphat.

2. Verfahren nach Anspruch 1, dadurch gekennzeichnet, daß der Ligand durch eine kovalente chemische Bindung an die Oberfläche des Basisprodukts gekoppelt wird.

3. Verfahren nach einem der Ansprüche 1 oder 2, dadurch gekennzeichnet, daß der Ligand unter Zwischenschaltung eines Raumarmes, wie des Restes eines heterobifunktionellen Reagens oder einer Gruppe von mehreren Zuckern, an die Oberfläche des Basisprodukts gekoppelt wird.

4. Verfahren nach Anspruch 3, dadurch gekennzeichnet, daß der Raumarm durch eine Gruppe von mehreren Zuckern, wie die Gruppe (1-4)-O-β-D-Glucopyranosyl-(1-6)-O-β-D-glucopyranosyl, gebildet wird.

5. Verfahren nach einem der Ansprüche 1 bis 4, dadurch gekennzeichnet, daß das Basisprodukt ein submikroskopisches Teilchen, wie ein Liposom oder ein Polymernanoteilchen, ist.

6. Verfahren nach einem der Ansprüche 1 bis 5, dadurch gekennzeichnet, daß das Produkt oder das Basisprodukt ein Molekül oder ein Makromolekül natürlichen oder synthetischen Ursprungs ist.

7. Verfahren nach Anspruch 6, dadurch gekennzeichnet, daß das Molekül oder das Makromolekül ein Asiatikosid, ein Digalactosyldiglycerid oder ein Neoglycoprotein ist, welches beispielsweise durch Kombination eines Serumalbumins mit einem genannten Zucker erhalten wird.

8. Verfahren nach einem der Ansprüche 1 bis 7, dadurch gekennzeichnet, daß das Basisprodukt eine Substanz von kosmetischem oder pharmazeutischem, insbesondere dermatologischem, Interesse ist oder enthält, wie ein(en) Hautzellenstoffwechselmodulator.

9. Verfahren zum Selektieren eines Produkts, das dazu bestimmt ist, an der Membran eines Keratinozyts mittels einer Ligand-Rezeptor-Bindung fixiert zu werden, dadurch gekennzeichnet, daß man aus einer Gruppe von Produkten jene auswählt, die durch ein Basisprodukt gebildet sind, das an mindestens einen durch einen für die Membranrezeptoren zugänglichen Osidrest gebildeten Ligand gekoppelt ist, wobei der Osidrest ausgewählt ist aus Rhamnose, Galactose oder Galactose-6-phosphat, vorzugsweise aus α-L-Rhamnose und α-D-Galactose-6-phosphat.

10. Verfahren nach Anspruch 9, dadurch gekennzeichnet, daß das Basisprodukt ein submikroskopisches Teilchen, wie ein Liposom oder ein Polymernanoteilchen, ist.

11. Verfahren nach einem der Ansprüche 9 oder 10, dadurch gekennzeichnet, daß das Produkt oder das Basisprodukt ein Molekül oder ein Makromolekül natürlichen oder synthetischen Ursprungs ist.

12. Verfahren nach Anspruch 11, dadurch gekennzeichnet, daß das natürliche oder synthetische Molekül ein Asiatikosid, ein Digalactosyldiglycerid oder ein Neoglycoprotein ist, welches beispielsweise durch Kombination eines Serumalbumins mit einem genannten Zucker erhalten wird.

13. Verfahren nach einem der Ansprüche 9 bis 12, dadurch gekennzeichnet, daß das Produkt oder das Basisprodukt eine Substanz von kosmetischem oder pharmazeutischem, insbesondere dermatologischem, Interesse ist oder enthält, wie ein(en) Hautzellen-Stoffwechselmodulator.

14. Verfahren zur Herstellung eines Produkts, das dazu bestimmt ist, an der Membran eines Keratinozyts mittels einer Ligand-Rezeptor-Bindung fixiert zu werden, dadurch gekennzeichnet, daß man ein entsprechendes Basisprodukt an mindestens einen durch einen für die Membranrezeptoren zugänglichen

Osidrest gebildeten Ligand koppelt, wobei der Osidrest ausgewählt ist aus Rhamnose, Galactose und Galactose-6-phosphat, vorzugsweise α-L-Rhamnose und α-D-Galactose-6-phosphat.

**15.** Verfahren nach Anspruch 14, dadurch gekennzeichnet, daß der Ligand durch eine kovalente chemische Bindung an die Oberfläche des Basisprodukts gekoppelt wird.

**16.** Verfahren nach Anspruch 14 oder 15, dadurch gekennzeichnet, daß der Ligand unter Zwischenschaltung eines Raumarmes, wie des Restes eines heterobifunktionellen Reagens oder einer Gruppe von mehreren Zuckern, an das Basisprodukt gekoppelt wird.

**17.** Verfahren nach Anspruch 16, dadurch gekennzeichnet, daß der Raumarm durch eine Gruppe von mehreren Zuckern, wie die Gruppe (1-4)-O-β-D-Glucopyranosyl-(1-6)-O-β-D-glucopyranosyl, gebildet wird.

**18.** Verfahren nach einem der Ansprüche 14 bis 17, dadurch gekennzeichnet, daß man als Basisprodukt ein submikroskopisches Teilchen, wie ein Liposom oder ein Polymernanoteilchen, verwendet.

**19.** Verfahren nach Anspruch 18, dadurch gekennzeichnet, daß das prozentuelle Molverhältnis der Osidreste zur Gesamtheit der die Doppelschicht des Liposoms bildenden Lipidmoleküle mindestens 10 %, vorzugsweise mindestens 15 %, bezogen auf die Gesamtheit der amphiphilen Lipide, ist.

**20.** Verfahren nach einem der Ansprüche 18 oder 19, dadurch gekennzeichnet, daß die Liposome derart formuliert werden, daß sie eine starke Mikroviskosität aufweisen.

**21.** Verfahren nach einem der Ansprüche 14 bis 17, dadurch gekennzeichnet, daß man als Basisprodukt ein Molekül oder ein Makromolekül natürlichen oder synthetischen Ursprungs, wie ein Protein, insbesondere ein Serumalbumin, verwendet.

**22.** Verfahren nach einem der Ansprüche 14 bis 21, dadurch gekennzeichnet, daß das Produkt oder das Basisprodukt eine Substanz von kosmetischem oder pharmazeutischem, insbesondere dermatologischem, Interesse, wie ein Hautzellenstoffwechselmodulator.

**23.** Verfahren nach einem der Ansprüche 14 bis 21, dadurch gekennzeichnet, daß das Produkt oder das Basisprodukt eine Substanz von kosmetischem oder pharmazeutischem, insbesondere dermatologischem Interesse enthält, wie einen Hautzellenstoffwechselmodulator.

**24.** Verfahren zur Herstellung einer kosmetischen oder pharmazeutischen, insbesondere dermatologischen, Zusammensetzung, dadurch gekennzeichnet, daß ein kosmetisch oder pharmazeutisch akzeptabler Exzipient, Vehikel oder Träger mit mindestens einem Produkt, wie dem durch das Verfahren nach einem der Ansprüche 9 bis 23 erhaltenen, vereinigt wird.

**25.** Verfahren nach Anspruch 24, dadurch gekennzeichnet, daß die Zusammensetzung für jede Art von Pflege oder Behandlung, die für Keratinozyten von Interesse ist, insbesondere zur Verbesserung der Hautregeneration, zur Behandlung von Psoriasis und für den Haarnachwuchs, bestimmt ist.

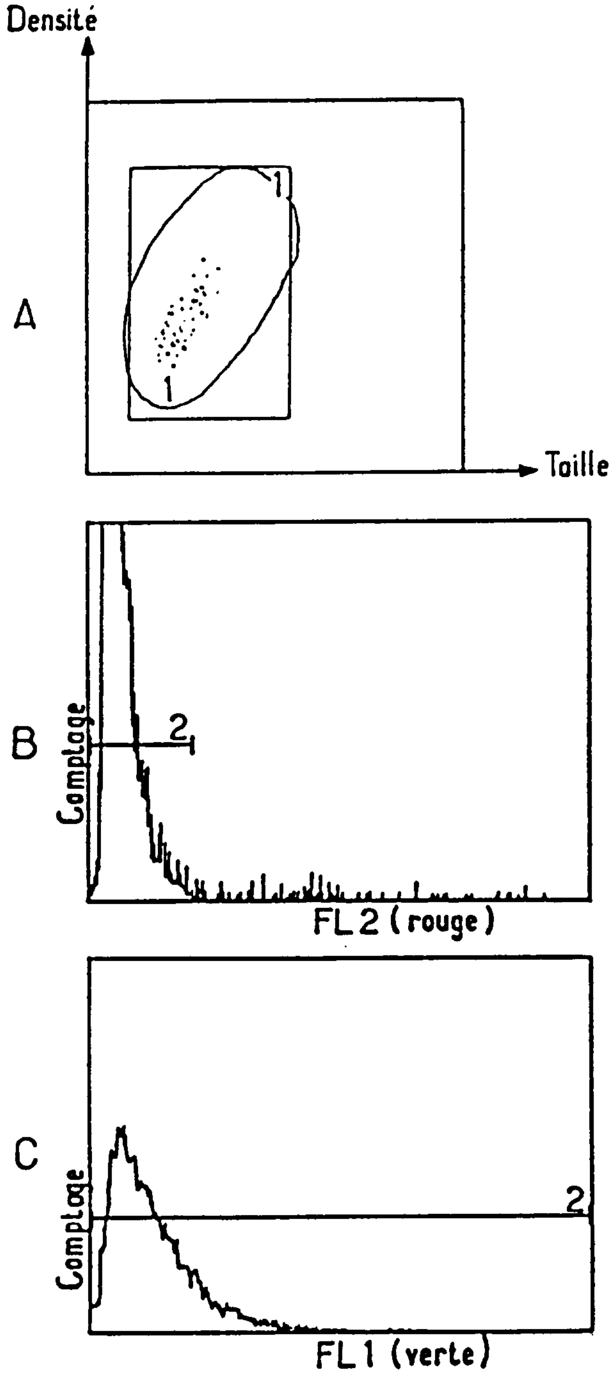
Densité
Taille
A
1
1
B
Comptage
2
FL 2 (rouge)
C
Comptage
2
FL 1 (verte)

FIG.1

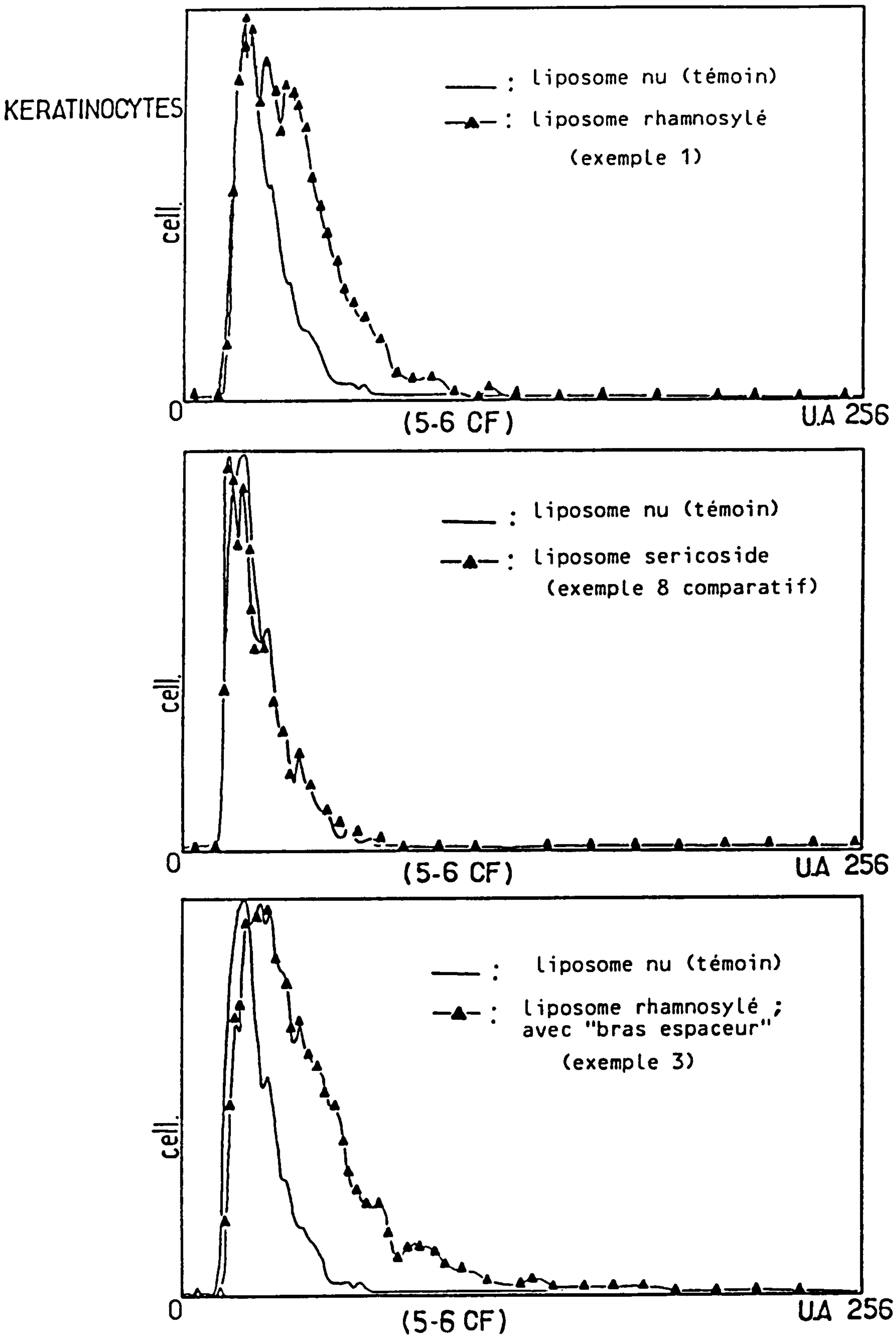
KERATINOCYTES
cell.
: liposome nu (témoin)
: liposome rhamnosylé
(exemple 1)
0
(5-6 CF)
UA 256
cell.
: liposome nu (témoin)
: liposome sericoside
(exemple 8 comparatif)
0
(5-6 CF)
UA 256
cell.
: liposome nu (témoin)
: liposome rhamnosylé ;
avec "bras espaceur"
(exemple 3)
0
(5-6 CF)
UA 256

FIG. 2

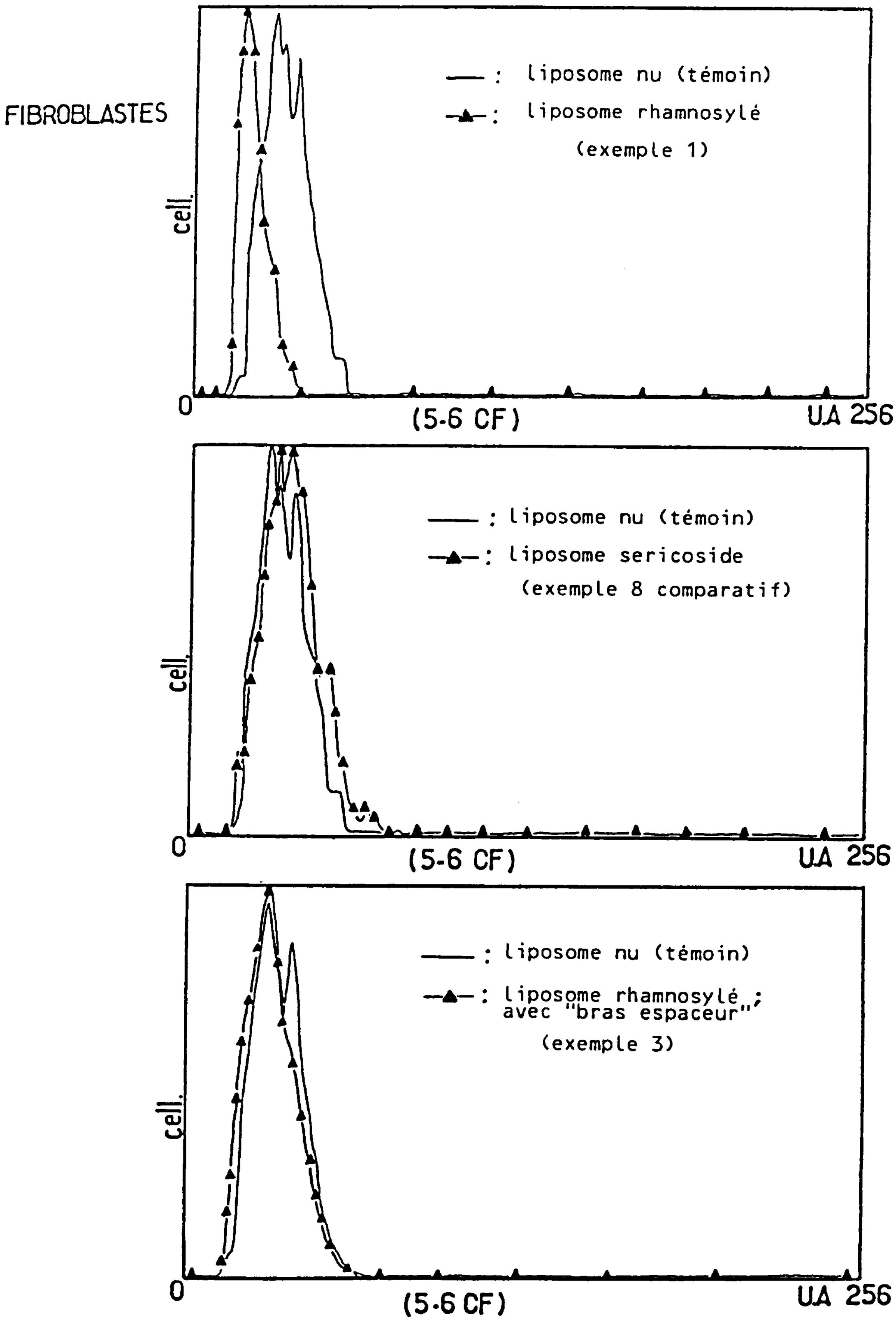
FIBROBLASTES
—— : liposome nu (témoin)
—▲— : liposome rhamnosylé
(exemple 1)
cell.
0
(5-6 CF)
UA 256
—— : liposome nu (témoin)
—▲— : liposome sericoside
(exemple 8 comparatif)
cell.
0
(5-6 CF)
UA 256
—— : liposome nu (témoin)
—▲— : liposome rhamnosylé ; avec "bras espaceur"
(exemple 3)
cell.
0
(5-6 CF)
UA 256

FIG.3